# EUROPEAN PATENT SPECIFICATION

(11) **EP 0 607 343 B1**
(45) Date of publication and mention of the grant of the patent: **13.03.2002**
(21) Application number: 92922478.0
(22) Date of filing: 09.10.1992
(51) Int. Cl.: A61M 5/00, A61M 5/14, F16K 7/04, F16K 7/06

(54) **ROTATABLE MEDICAL VALVE CLOSURE**
ROTIERBARER, MEDIZINALER VENTILVERSCHLUSS
DISPOSITIF MEDICAL D'OBTURATION A SOUPAPE

(30) Priority: 11.10.1991 US 776581
(43) Date of publication of application: 27.07.1994
(60) Divisional application: 01118691.3
(73) Proprietor: BOSTON SCIENTIFIC CORPORATION, Natick, MA 01760-1537 (US)
(72) Inventor: CHU, Michael, S., H., Brookline, MA 02146 (US); CHIN, Yem, Burlington, MA 01803 (US); CRAGG, Andrew, Edina, MA 55424 (US); GELLMAN, Barry, N., North Easton, MA 02356 (US)
(74) Representative: Wallace, Sheila Jane
(86) International application number: US9208687
(87) International publication number: WO9306877

(56) References cited:
- WO-A-93/06878
- GB-A- 745 844
- US-A- 2 987 292
- US-A- 3 920 215
- US-A- 4 378 013
- US-A- 4 540 411

## Description

This invention relates to closure devices, for example through-channel valves and the like for medical applications, in particular, for the control of fluids and devices passing in or out of patient's body. Operation of prior devices to some extent has been awkward and required more hand actions and the presence of more personnel than at times has been desirable.

Catheters are placed inside blood vessels and body cavities, typically by being slid over guidewires. During placement, some way is needed to prevent backflow of blood or other fluid from the proximal end of the catheter. Many times it is also desirable, with minimum steps and effort, to attach a hand syringe to the proximal end of the catheter, open the catheter valve, inject fluids through the catheter and then remove the syringe and close the system. Many other applications require closure of a through-passage to, for example, control the flow of fluids as by a stopcock or to grip a device, such as a guidewire, to provide a handle to enable working the wire to achieve its insertion and accurate placement in the body.

While it is well known to use luer fittings for attachment of medical instruments, e.g. a syringe to a catheter, valves controlling flow that use luer fittings generally do not have through-passages and also, for the most part, require separate activating motions.

The attachment of an external instrument such as a syringe to the passageway of a closure device in a manner that also opens the closure device is also known, but in a form that has had significant limitations. Such a device, for example, is described in U.S. Patent No. 4,243,034. The described device relies on an axial sliding motion for making the attachment and opening a valve by releasing one or more balls from depression into the wall of a resilient tube. This device is neither as simple nor as effective as desired by those in the field, and also fails to meet all needs for single-handed operation in flow-control devices.

Other known closure or wire-gripping devices provide partial benefits but fail to combine all desirable features in one unit. For example, one type of device, known as a Tuohy-Borst closure, comprises a cap which, when screwed on, axially compresses a captured thick-walled tubular segment (a grommet) to seal a passage through the tubular segment. This device is disadvantageous in that it requires many turns to seal the passage.

US Patent 4 540 411 discloses a catheter placement device in which a pair of relatively rotatable valve members are connected to the catheter. An elastomeric longitudinally extending tubular member is connected between the valve members and is twistable in response to relative rotation of the valve members. An introducer needle extends longitudinally through the tubular member and catheter for introducing the distal end of the catheter into a blood vessel. Predetermined relative rotation between the valve members effects twisting of the tubular member about the needle and closure of the tubular member. A side port may be connected to a valve member for introducing fluid into a patient.

British Patent Specification No: 745 844 discloses a valve comprising a rigid tubular body which may be part of an existing fitting. A rubber or like elastic flow-tube is frictionally or otherwise located in the bore of the tubular body. It is normally in close contact with the whole periphery of the bore. A ball or other plunger is mounted in a hole of the body wall opposite the position of this flow tube. A ring or sleeve is rotatable upon the exterior of the tubular body so as to cover the plunger. The inner periphery of the ring or sleeve has a cam surface which cooperates with the plunger so that the latter is caused to deform the flow tube to a greater or lesser degree according to the direction in which the ring or sleeve is turned.

US Patent 3 920 215 is concerned with what is described as a compact and simple valve suitable for irrigation systems which makes effective use of low cost injection moulded parts. The valve includes a sleeve of resilient material and a housing surrounding the sleeve. The housing has a pair of guides. A pair of closing members are slidably mounted in the guide. A cam is rotatably mounted about an axis coincident with the axis of the tube to move the closing members together to pinch the tube closed.

US Patent 2 987 292 is concerned with a mechanically operated collapsible valve suitable for use in handling corrosive fluids in refineries and in the chemical industry.

The valve has a tubular body with an axially extending fluid flow bore and three side openings equally spaced about the bore. A flexible bore liner extends across these openings sealing off access of fluid in the bore to the openings. Three inserts at the outside of the openings are sized to enter the bore through the openings. These inserts have equal circular cross-sections in planes normal to the bore axis. The valve also has a rotary actuator including cam means rotatable in one direction about the tubular body in camming engagement with the inserts to cause the inserts to enter the bore simultaneously and to equal progressive extent. They thus displace the liner at three symmetrical locations for reducing the fluid flow bore area. The rotary actuator is operable when rotation is stopped to hold the inserts at any one of an infinite number of selected positions of equal progressive entrance into the bore. The curved surfaces of the inserts in the valve closed position positively hold the bore liner in Y-shaped cross-sectional configuration and exert compressive loading against opposite sides of the Y-arms of the bore liner when the inserts are fully extended into the body.

In accordance with the invention, we provide a closure device to which a cooperative component is attachable for communication therebetween, said closure device comprising a rotatable body portion including a connector for connection to and disconnection from a mating connector of the cooperative component by relative rotation between said rotatable body portion and the cooperative component, and a relatively stationary body portion of said closure device; said rotatable body portion being rotatable relative to said stationary body portion between an open position in which a passage is defined through said closure device and a closed position in which said passage is closed; the closure device being characterised in that said body portions are constructed and arranged such that the direction of relative rotation of said connectors for connection of said rotatable body portion to said cooperative component corresponds to the direction of rotation of said rotatable portion relative to said stationary body portion toward said open position to also open said passage and the direction of relative rotation of said connectors that disconnects said rotatable body portion from said cooperative component corresponds to the direction of rotation of said rotatable body portion relative to said stationary body portion toward said closed position to also close said passage.

The above recited features of the invention may be related to specific features of particular described embodiments as follows:

There is described hereinbelow embodiments of closure device (2) to which a cooperative component (8) is attachable for communication therebetween, said closure device comprising a rotatable body portion (4) including a connector (6) for connection to and disconnection from a mating connector (7) of the cooperative component (8) by relative rotation between said rotatable body portion (4) and the cooperative component (8), and a relatively stationary body portion (12) of said closure device. The rotatable body portion (4) is rotatable relative to said stationary body portion (12) between an open position in which a passage (20) is defined through said closure device and a closed position in which said passage (20) is closed, said body portions being constructed and arranged such that the direction of relative rotation of said connectors for connection of said rotatable body portion (4) to said cooperative component (8) corresponds to the direction of rotation of said,rotatable portion (4) relative to said stationary body portion (12) towards said open position to also open said passage (20), and the direction of relative rotation of said connectors that disconnect said rotatable body portion (4) from said cooperative component (8) corresponds to the direction of rotation of said rotatable body portion (4) relative to said stationary body portion (12) towards said closed position to also close said passage (20).

It should, however, be understood that the reference numerals set out in the aforementioned paragraph are provided for the purpose of explanation only and that no limitation is intended or implied.

We describe below embodiments of device for closure of a through-passage for use, for example, as a valve for a catheter or as a gripping member for a device such as a guidewire placed in the through-passage or channel. Our described closure devices are of a simple, rugged, inexpensive and durable construction that will not inadvertently disassemble by the activating motions. Our embodiments of closure device improve upon the operation and configuration of prior closure devices for attachment and detachment to medical components, such as the attachment of a syringe to a catheter.

In the embodiments described rotation of a rotary cam displaces a compression member against the side of a resilient tube. Such a closure device may be constructed for both hand rotation and rotation by syringe, and the direction of rotation is the same for both attaching the syringe and opening a valve of the closure device.

The closure device can be provided as a clamping or sealing device for clamping a guidewire or a catheter, operable in combination with an introducer sheath to prevent backflow of blood before or after insertion of the catheter or guidewire through the sheath; and by a kit comprising a vena cava (e.g., Greenfield) filter, a stabilizer, a surrounding elongated placement catheter and an introducer sheath for insertion of the filter into the body.

The device may be arranged to be opened by connecting motions of a syringe or the like, and closed by the reverse motion of disconnecting the syringe.

Thus, the medical closure device may control fuids or objects passing into or out of the body or the like. A through-passage having an axis may be defined by a resilient member. An internal cam may be provided in the relatively stationary body member, having a cam surface oriented about and spaced from the axis of the passage with a first surface portion disposed relatively closer to the axis than a circumferentially spaced second surface portion. A compression member positioned in a radially extending aperture in the rotatable body portion is biased radially outward by the resilient member to maintain contact with the cam surface. Adjustment of the radial compression of the resilient member for closing or opening the passage is by relative rotation of the body portions to position, in dependent manner, the compression member radially closer to or further from the axis.

Preferred embodiments have one or more of the following features. The combination is constructed so that the rotational force required to rotate the body portion to effect change in the compression of the resilient member to open and close the passage is less than the rotational force needed, respectively, to complete the connection and disconnection of the cooperative component relative to the closure device. Preferably, the body portion is constructed to define a rotational luer lock fitting for interfitting with a matching fitting on the cooperative component. Also preferably, the rotatable body portion is threadably attached about the axis to a second relatively stationary body portion, the threads thereof being of opposite hand to threads of the rotatable connection between the closure device and the cooperative component, whereby rotation to connect together the cooperative components with the device causes the body portions to move apart. Preferably also, a stop surface is defined at the end of the second cam surface portion, arranged to engage the compression member and stop relative motion apart of the body portions when the compression member reaches passage-opening position, and the first and second body members are constructed to engage each other and stop threaded-together motion when the compression member reaches passage-closing position, the stops cooperating to enable positive connecting and disconnecting actions between the cooperative component and the closure device.

Further preferred embodiments have one or more of the following features.

The device is constructed for use as a medical device in a flow path for fluid passing into or out of a living body wherein the proximal end of the device, relative to the living body, is constructed to be connected to a cooperative component in the form of a syringe or other injection device. Preferably, the distal end of the closure device relative to the living body is constructed to be connected to an angiographic catheter. Preferably also, the closure device further includes a side entry channel distal of the axial position of the compression member.

Preferred embodiments have one or more of the following features.

A stationary first body portion fixes the position of the tubing and a second body portion, carrying the cam surface, is mounted to rotate upon the first body portion. The tubing is preferably comprised of a silicone elastomer.

The first surface portion of the cam positions the compression member radially to completely close in fluid-tight manner the through-passage, and the second surface portion positions the compression member to fully open the through-passage for fluid flow.

The cam defines a smooth, spiral-form surface. Preferably, the compression member is a single ball-form member engaged upon the spiral surface. Also preferably the rotatable body portion is axially and rotatably positioned by a screw thread.

The rotatable body portion includes a first stop member which is engaged by the compression member preventing disassembly of the body portion by blocking further rotation. Preferably, the compression member is held axially fixed with respect to the tubing member, and the first stop member is a stop surface on the rotatable body portion at the end of the second portion of the cam surface for preventing rotation beyond the stop.

Also preferably, the rotatable body portion is positioned by a screw thread and the cam defines a spiral-form surface with an abrupt transition from the end of the second cam surface portion, furthest from the axis, to a portion of the body member closer to the axis, in a manner interacting with the compression member to permit continual rotation during assembly of the body portion in a screw-on direction in which the compression member is gradually, progressively compressed against the resilient member to the maximum at which state it remains until further rotation causes the second most furthest spaced portion of the cam surface to register with the compression member to permit a springing outwardly of the compression member to allow repetition of the rotational motion, whereas opposite rotation of the rotational body portion along the threads in screw-off direction is limited by the stopping of the compression member against the abrupt formation of the rotatable body portion.

Preferably also, a second stop member is provided for preventing rotation of the body portion beyond the first surface portion of the cam. Where the rotatable body portion is positioned by a screw thread, the first stop is preferably arranged adjacent to the second surface portion of the cam, furthest from the axis, upon which contact with the compression member is made to prevent further rotation in screw-off direction, and the second stop surface prevents rotation of the body in screw-on direction beyond the point at which the first surface portion of the cam, closest to the axis, is engaged with the compression member. Preferably, the second stop blocks further axial advance along the thread of the rotatable body portion. Preferably, the compression member is axially restrained in fixed position, relative to the resilient member, and a stationary body portion holds the resilient member in a fixed relation and defines a radially extending aperture in which the compression member is axially confined and is permitted to move radially against the resilient member.

Further preferred embodiments have one or more of the following features.

The tubing member is compressed axially and confined radially for sealing engagement of surfaces at its proximal and distal ends.

The body of the closure device and its through-passage are sized for passage therethrough of a guidewire providing, when the device is closed upon the guidewire, means for gripping and torquing the guidewire.

The body is attachable to tubing members for controlling the flow of fluid therethrough.

The rotatable body portion has an external surface exposed for engagement and rotatable operation by the hand of a user.

The closure device is constructed so that rotation of the rotatable body portion to open and close the through-passage is substantially one-half turn from stop to stop in opposite directions.

For control of a fluid at a pressure selected from over a wide pressure range, the thickness and character of the material of the resilient tubing member of the device is selected to withstand the selected pressure.

For control of a fluid at a flow rate selected from over a wide flow range, the inside diameter of the resilient tubing member is selected to accommodate the selected fluid flow rate.

The resilient tubing member and overlying body portion comprise clear plastic material enabling visual examination of flow through the through-passage.

The first and second body portions are formed of moldable material.

The closure device is preferably embodied as a medical closure device for providing a passage into or out of a medical cooperative component, the medical cooperative component being adapted to be inserted into a human or animal body, and the medical closure device providing passage for both pressurized fluids and a medical device, the connector of the rotatable body portion of the medical closure device being adapted for connection into and disconnection from a mating connector of the medical cooperative component.

Preferably, the medical closure device also comprises a resilient member at least partially defining the passage, the passage having an axis and being arranged for passage therethrough of a selected medical device and pressurized fluid, the resilient member being of material selected, in response to radial pressure applied to said resilient member, to form a pressure seal upon the medical device when the medical device is present in the passage and upon an opposing surface defining the passage when the medical device is not present; a proximal body portion including first and second members, the first member having a pressure fitting in sealing enegagment with and mounting the resilient member and the second member being disposed about and supporting the resilient member; a distal body portion including an internal cam having a cam surface oriented about and spaced from the axis of the passage with a first surface portion disposed relatively closer to the axis than a circumferentially spaced second surface portion; and a compression member positioned in a radially extending aperture in the proximal body portion and biased radially outwardly by the resilient member to maintain contact with the cam surface.

Preferably, the proximal and distal body portions are connected together in a manner permitting their relative rotation about the axis, the body portions and the resilient member being sealed together in a manner to withstand, without leakage, fluid under pressure in the passage. Preferably, also, the connected body portions are cooperatively related, whereby, upon relative rotation of the body portions, the cam surface is moved relative to the compression member to cause radial displacement of the compression member closer to the axis of the passage to cause the resilient member to grip and pressure seal upon the exterior of the medical device, when present in the passage, or upon the opposing surface defining the passage when the medical device is not present.

Other features and advantages will be apparent from the following detailed description of preferred embodiments taken together with the accompanying drawings wherein:
Figure 1 is a perspective illustration of a user holding the distal part of the closure device while connecting a syringe to the proximal part of the closure by turning;
Fig. 2 is a longitudinal cross-sectional view of the medical closure device of Fig. 1 in the open position, while Fig. 2A is a transverse cross-section of Fig. 2 along line A-A;
Fig. 2B is a longitudinal cross-sectional view of the closure device of Fig. 1 in the closed condition, while Fig. 2C is a transverse cross-section of Fig. 2B along line B-B;
Fig. 3 is an exploded view of the closure device of Figs. 1 and 2;
Figs. 4-4G illustrate steps in the assembly of the device, where Figs. 4-4B are longitudinal cross-sectional views and Figs. 4C-4G are a series of cross-sections, similar to Fig. 2A, taken through the center of the compression member at a sequence of rotary positions of the proximal body portion during assembly;
Figs. 5-5C taken in the direction of Line C-C in Fig. 2 illustrate gradual closure of the through-passage during operation showing positions of the cam surface and the radially moveable compression member;
Figs. 6 to 6E illustrate use of the device in a catheterization operation;
Figs. 7 and 7A illustrate an alternative embodiment of the device with an angled side arm for access to the through-passage distal of the point of closure;
Figs. 8 and 8A illustrate an alternative embodiment of the device with a 90 degree side arm entry for access to the through-passage;
Figs. 9 and 9A illustrate use of the device as a gripping member with its attachment to a guidewire;
Fig. 10 is a perspective illustration showing combination of a larger closure device according to the invention with an introducer sheath and use of the device as a gripping and closure control device in introducing a catheter, (containing a vena cava filter) through the device and sheath, into the vena cava of a patient;
Fig. 11 is a view in longitudinal cross-section of a catheter introducer assembly comprising a closure device, attached introducer sheath and dilator within the sheath;
Fig. 11A is a view similar to Fig. 10 of the introducer sheath of Fig. 11 through which a placement catheter containing a vena cava filter and stabilizer assembly extends;
Fig. 12 illustrates in longitudinal cross-section the device of Fig. 11 modified to have an angled side arm access port connected to the through-channel distal of the point of closure;
Fig. 13 illustrates in longitudinal cross-section the device of Fig. 11 modified to have a 90 degree side arm access port connected to the through-channel distal of the point of closure;
Figs. 14 and 15 show in cross-section along line A-A of Fig. 13 relative positions of the compression member and the resilient tubing in open and closed positions, respectively;
Figs. 16-17 show in cross-section the device of Fig. 11 with details of the vena cava filter and stabilizer within the placement catheter as they are inserted through the closure device and introducer sheath;

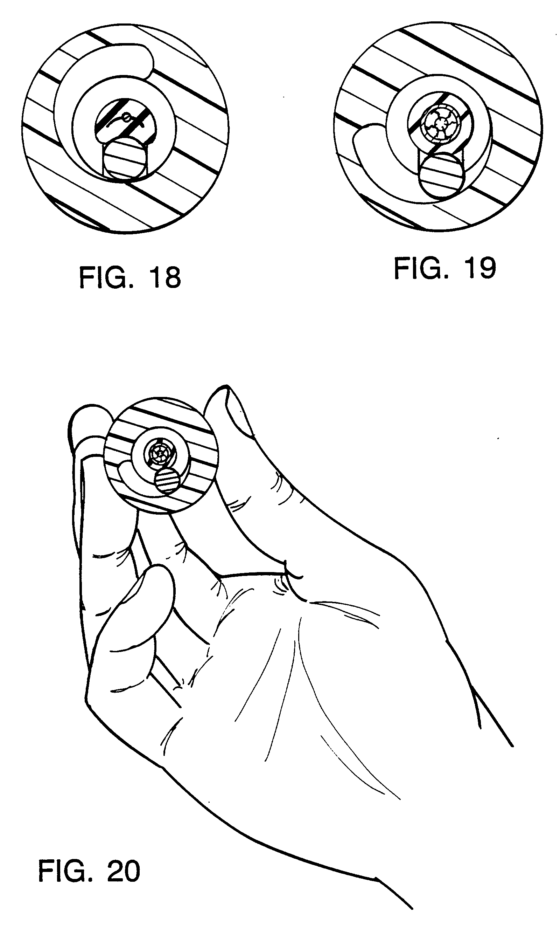

Fig. 18 shows in cross-section along line A-A of Fig. 16 the positions of the cam and other components in clamping and sealing relationship upon a guide wire while Fig. 19 is a similar view showing the cam in partially closed position in sliding/sealing relation to the filter introducer catheter during insertion of the introducer catheter into the body;
Fig. 20 illustrates relative size of the device in cross-section by depicting it being held by a user who is adjusting the closure by one hand;
Fig. 21 shows a device according to an alternate preferred embodiment;
Figs. 21A and 21B show cross-sectional views of the device of Fig. 21, taken along lines A-A and B-B, respectively;
Fig. 22 shows a reduced exploded view of the device of Fig. 21; and
Fig. 22A shows a partially assembled view of the device of Fig. 21.

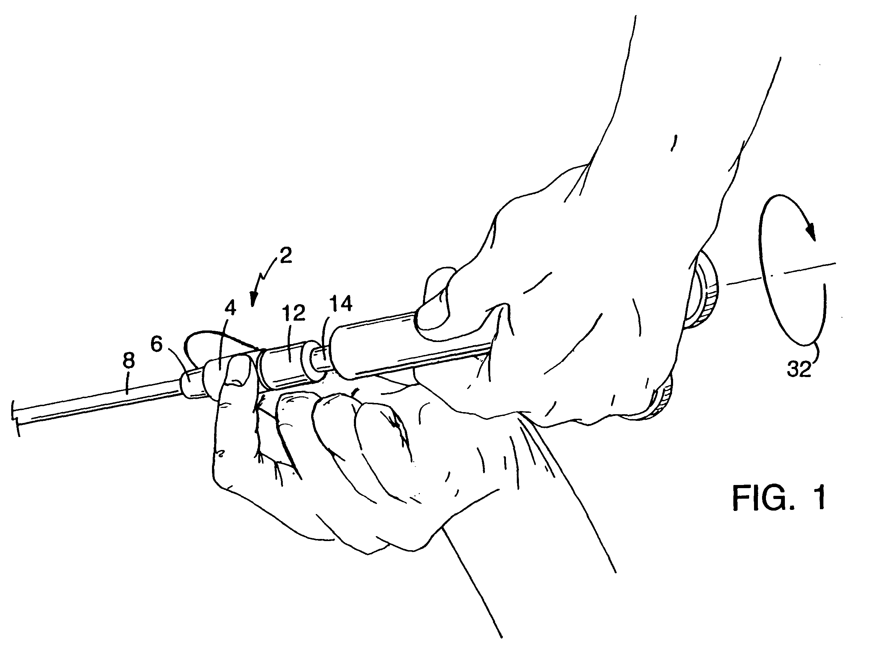

Referring to Figs. 1 to 3, medical closure device 2 includes a distal body member 4 having a distal end 6 configured as a male luer locking member for attachment, for example, to the hub 7 of an angiographic catheter 8 (5 French), and a proximal end 10 which is threadably (threads 13) attached to a proximal body member 12. The proximal body member includes a proximal end 14 which is adapted as a threaded (threads 15) female locking luer attachment and a distal end 16 that includes threads 13'. Overall length of this embodiment is about 3 cm and diameter about 1/2 inch (1.27 cm) as described below.

The external cylindrical surface of the body member provides a convenient gripping surface for hand operation of the closure device.

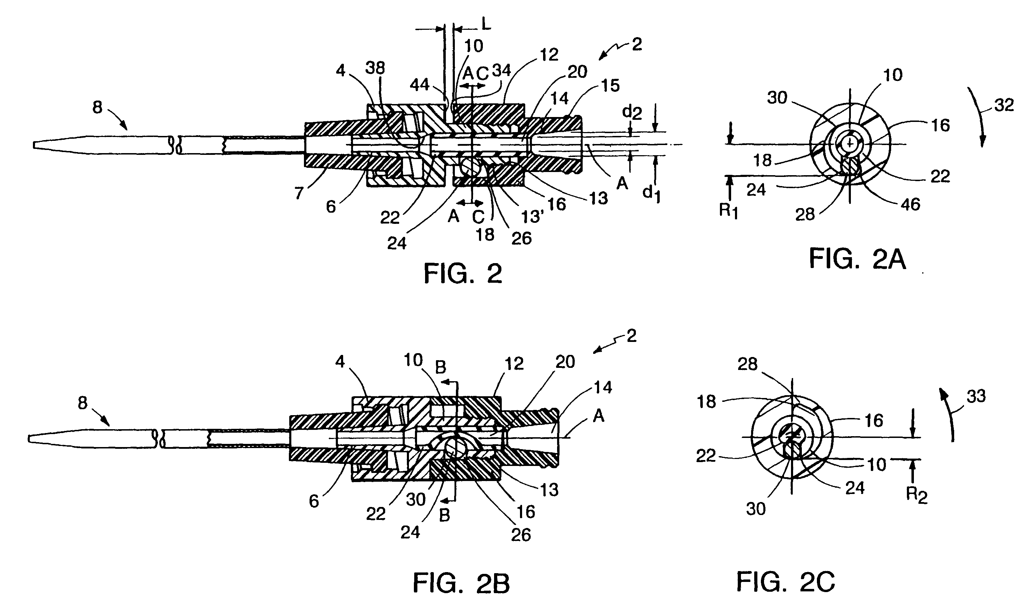

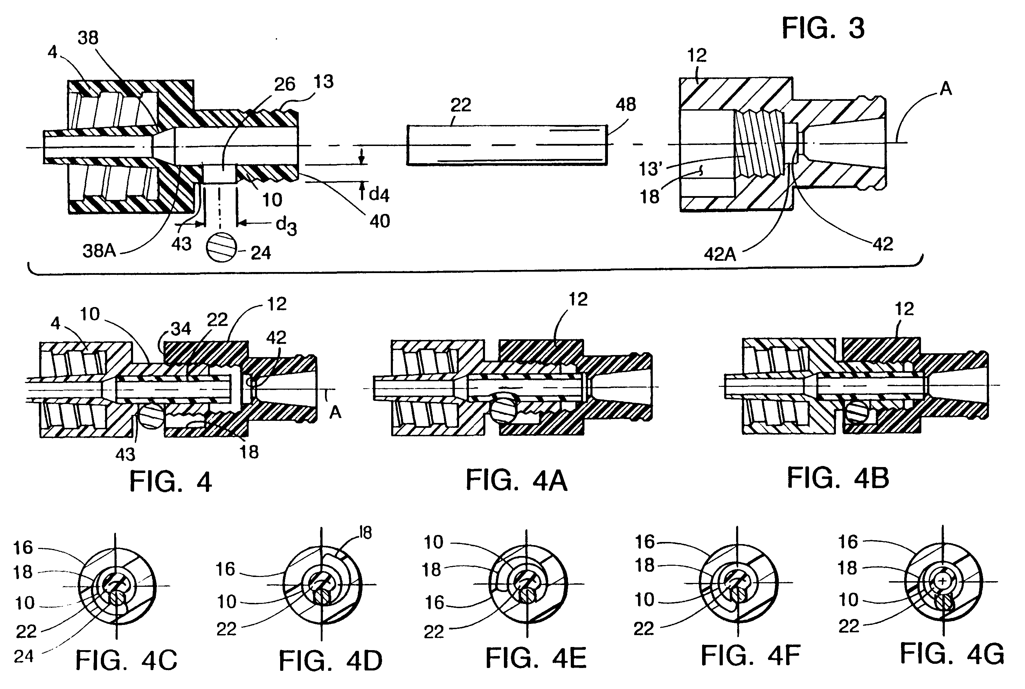

The distal end of the proximal body includes in its interior an inner cam 18 having a cam surface that is circumferentially arranged about axis A of the device. Channel 20, having an axis A, extends through the device (Fig. 2). A tubing member 22, preferably silicone tubing, is positioned in the channel. In this preferred embodiment, the silicone tubing has, in unstressed condition, an outside diameter d₁ of approximately 0.150 inch (0.381cm), an inside diameter d₂ of .060 inch (0.1524 cm), and a wall thickness of about .045 inch (0.11432 cm). When installed in the proximal end 38A of the distal body member and the distal end 42A of the proximal body member (see Fig. 3), the tubing is compressed and supported by the walls of the body so that it, in open condition, has an outside diameter of 0.141 inch (0.35814 cm) and an internal diameter of 0.055 inch (0.1397 cm).

A valve is formed by a spherical compression member 24, preferably a stainless steel ball with a diameter of about 0.125 inch (0.3175 cm), which is biased radially outward by tubing member 22 such that the compression member maintains contact with cam 18. As illustrated particularly in Fig. 3 compression member 24 is positioned in an aperture 26 in the proximal portion 10 of distal body 4 to prevent axial motion of the compression member, but permit and guide its radial movement. Preferably, the aperture has a diameter d₃ of .128 inch (0.32512 cm), and a depth d₄ of 0.056 inch (0.14224 cm).

To effect opening of the through-passage, the relative rotation of distal body 4 and proximal body 12 in direction 32 is adjusted such that the compression member is positioned along portion 28 of cam surface 18, furthest in radial position, distance R₁, from axis A, as illustrated in Figs. 2 and 2A. The cross-sectional views of Figs. 2A and 2C illustrate that when rotated clockwise from the closed position (Fig. 2C), the movement of the proximal body member causes the compression member to disengage its compression of the tubing in a linear fashion by action of the cam surface, until a point is reached when the compression member no longer compresses the tubing and is positioned on cam surface portion 28. The compression member then comes into abutment with butt stop 46 (Fig. 2A) formed by the proximal body, preventing further rotation.

Similarly, to effect closure of the through-passage, proximal body member 12 is rotated counter-clockwise relative to distal body member 4 (arrow 33 on Fig. 2C) causing compression member 24 to be positioned on portion 30 of the cam which is closest, dimension R₂, to axis A of the through-passage. This moves the compression member radially inward to compress the tubing member 22 and reduce and close the opening of the through-passage, as illustrated in Figs. 2B and 2C.

Left-handed threading 13 and 13' is used in joining the distal and proximal body portions. Therefore, as proximal body member 12 is rotated in a counterclockwise direction, as viewed from the proximal end of the closure device (in the "screw-on" direction, indicated by arrow 33), the proximal body member moves axially distally, i.e., closer to distal body member 4. The cam is oriented such that full closure of the free passage is reached at a point of the counter-clockwise rotation when surfaces 34 and 44 of the proximal and distal body-members, respectively, butt with one another, giving the feel also of a definite stop point for the fully closed position.

Thus, in this embodiment, in which the closure device is used as a stop cock with a syringe attachment, the cam is oriented such that opening of the through-passage is effected by clockwise rotation and closure of the through-passage is effected by counter-clockwise rotation of the proximal body member, as viewed from the proximal end of the device. This arrangement advantageously allows the user to attach the syringe and open the passage, or close the passage and remove the syringe, with a single motion.

The axial travel of the proximal body member between the fully open and fully closed positions (Figs. 2A and 2C) is indicated by gap "L" (Fig. 2). In the preferred embodiment, this distance is about .030 inch (0.0762 cm) and is covered by an approximate 180° rotation of the proximal body member. During rotation, the portion of the cam surface contacting the ball-form compression member lies at a radial distance from the axis A that changes by a similar dimension, .030 inch (0.0762 cm) between open and closed positions.

Luer connector 14 (with Luer taper from dimension d₁ to d₂ and external Luer threads) is configured in the common manner, to require a clockwise rotation of an external component, such as a syringe, for attachment to the closure device 2. With this rotation arrangement and the closure device in the closed condition initially, the proximal body member resists relative rotation when the syringe is being attached because the tubing member under compression by the compression member produces frictional drag. The syringe thereby can be initially lead on to the luer thread. The resistance of the cam arrangement is overcome when the component becomes partially engaged on luer connector 14 by the clockwise rotation and resistance of the luer connection increases as normally occurs. At that point, further clockwise rotation of the syringe causes simultaneous clockwise rotation of the proximal body portion, and channel 20 is opened as the compression member moves along cam surface to cam portion 28. When the compression member reaches the butt stop 46, the proximal body portion is stopped from rotation, and further clockwise rotation of the syringe enables the luer connection to reach its final locked position.

When, after use, the syringe is to be removed, the syringe is subsequently rotated in the counter-clockwise direction. The proximal body portion 12 rotates simultaneously with the syringe, because of the locking friction of the luer in its locked position, (there is also less resistance exerted on cam surface 18 by compression member 24 when the tubing, in open position, is not significantly compressed). After the proximal body portion has been rotated such that compression member 24 is moved into contact with cam portion 30 and the through-passage is closed, surfaces 44 and 34 of the two body members move into abutment by the left-handed mounting threads, establishing a definite stop point indicative of the closed condition of the through-passage. The syringe is then disconnected by its further rotation in the same direction to overcome the resistance of the luer lock.

In an embodiment where the closure device is used with a syringe as a connected component, the device preferably has an outside diameter of about 0.445 inch (1.1303 cm) and is approximately 3 cm in length. A typical syringe may have an outside diameter of 0.75 inch (1.905 cm) and length of 10 cm. A particular advantege of the preferred embodiment incorporating the described rotary cam/ball valving arrangement described is its dual operability, i.e., its ability to be opened or closed either by hand or by attachment of an external component such as a syringe.

Another particular advantage is that, once assembled, the simple construction of the device prevents its disassembly. Clockwise rotation of the proximal body member 12 causes the cam surface to ride along the compression member to effect opening of the through-passage. When the relative rotation of the distal and proximal body members places portion 28 of the cam surface in contact with compression member 24, further rotation is prevented by butt surface 46 which engages the axially-stationary compression member. This prevents further clockwise rotation and any disassembly of the device in this direction.

Similarly and conversely, when the relative rotation is such that the compression member 24 or ball is adjacent to cam surface portion 30 closest to axis A, end surface 34 of the proximal body member engages surface 44 of the distal body member, thereby preventing any further rotation and possible damage to the compression member or body members.

With rotation being thus limited in either direction, disassembly of the device by rotation of threads 13 and 13' is entirely prevented.

This feature of having a device of few and rugged parts that is incapable of being disassembled is of particular importance since it makes the device virtually fail-safe because it can neither be overtightened nor disassembled which could lead to loss of parts, introduction of dust, blood, etc.

Preferably, markings are on the body of the closure device so that the operator can tell whether the device is in the ON or OFF position and know the direction for rotation to the other setting.

Referring to Fig. 3 as well as Figs. 4 to 4G, assembly of an embodiment of the device is illustrated.

Assembly of the distal body member with the proximal body member involves an interference fit of the elastomeric tube with the two slightly tapered female connections, one on the proximal end 38A of the distal body member 4 and one on the distal end 42A of the proximal body member 12. The significant taper 38 of channel 20 serves to accomodate the thickness of the tube, to prevent tube movement while allowing for variation in the length of the tube, and to facilitate insertion of a guidewire or catheter through the channel. The taper of the ends of the channel in each body portion which the tube ends enter for their interference fit are tapered only slightly for achieving the desired interference. The tubing is first inserted into the channel of one member using a drop of silicone oil on the tube during assembly. This facilitates the joinder while also assisting in the combined radial and axial compression sealing of the tubing within the channel. Although oil is not needed on the compression member and in the recess within which it operates, some of the oil from the tubing may come into contact with the compression member which presents no problem and can benefit smooth operation of the closure device.

The tubing, due to the surrounding support provided by the housing walls that define channel 20, is capable of withstanding fluid pressures of greater than 1000 psi (6.895 x 10⁶N/m²). Thus, the device may be used for example, to handle pressures of 300-400 psi (2.068 to 2.758 x 10⁶N/m²) where a contrast media is to be injected rapidly from a syringe, and pressures up to 1,050 psi (7.239 x 10⁶N/m²) when used with a contrast media injector. High injection pressure is required in such an application so that the contrast media is rapidly totally injected and thus it is not dispersed. The pressure is needed to overcome the flow resistance of the relatively long path it must travel to reach the point in the body for the intended fluoroscopic medical observation.

In assembly, proximal body 12 is aligned with, and partially threaded onto, the proximal portion 10 of distal body 4 such that end surface 34 is roughly in alignment with the edge of aperture 26 in which the compression member is placed (Fig. 4). Tubing member 22 has at this point already been positioned in the distal body member 4. The length of tubing member 22 (overall length about .480 inch - 1.2192 cm) extends from a taper 38 in distal body 4 to beyond (about 3.0 mm) the most proximal end 40 of the distal body member 4, so that when assembly is complete, the proximal end of tube 48 reaches surface 42 to position the tube, though the main scaling effect is achieved by interference with the taper of the associated body portion into which the proximal end of the tube fits. Use of the resilient tubing in this manner produces both a fluid-tight and an air-tight seal, and allows operation with the high fluid pressures mentioned.

In the next step of assembly, compression member 24, here in the form of a ball, is positioned in aperture 26 (Fig. 4). The compression member's diameter is larger than depth d₄ of the aperture on the proximal portion of the distal body member, so the compression member will naturally extend beyond the outer edge 43 of proximal body portion 10 of distal body 4, being biased outwardly by the resilient tubing member 22 (Fig. 4). The compression member also extends beyond cam surface 18, even with the cam surface rotated to a position radially furthest from axis A, i.e. in open position.

To proceed with assembly, the compression member is then depressed radially, for example with the finger, so that it does not extend beyond surface 18 and, simultaneously, proximal body portion 12 is rotated in the "screw-on" direction counter-clockwise a number of turns such that its distal end overlaps the compression member (Fig. 4A). Whereas the rotatable body portion defines a spiral-form cam surface with an abrupt transition from the end of the cam surface portion 28, furthest from the axis, to a portion of the body member closest to the axis, continual rotation during assembly of the body portion in screw-on direction is permitted by the parts. The compression member is gradually progressively compressed against the resilient member until the maximum is reached at which state it remains until sufficient further rotation causes the abrupt transition and surface portion 28 of the cam surface to register with the compression member, (Fig. 4B) and permit spring outwardly of the compression member to permit repetition of the rotational motion, (whereas opposite rotation of the rotatable body portion along the threads is limited by stopping of the compression member against the abrupt formation, butt surface 46, of the rotatable body portion). Figs. 4C through 4G show various positions of the cam surface relative to the compression member during the assembly stages. As discussed, because of stop surfaces 34, 44, 46 (Figs. 2 and 2A), the device, once assembled, cannot be disassembled by rotation in either direction.

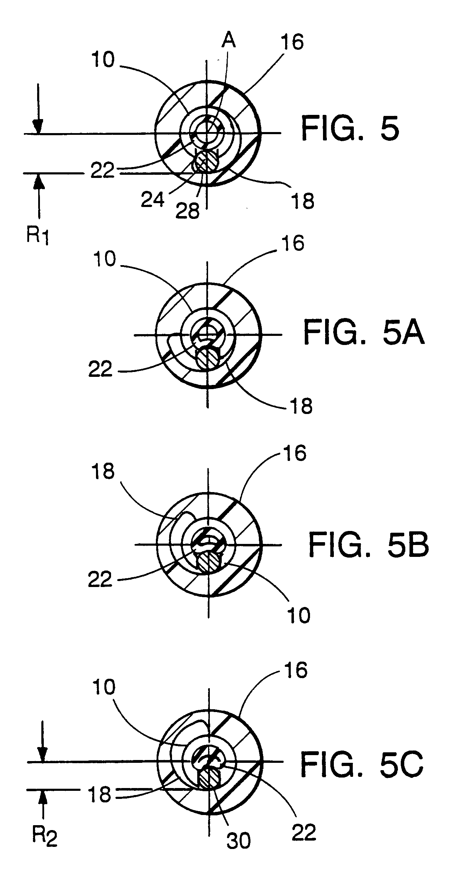

Continuous gradual control of the size of the through-passage is illustrated in the series of Figs. 5 to 5C, which show, in cross-section taken along line C-C of Fig. 2, cam surface 18, compression member 24, conformable tubing member 22 and axis A. As illustrated, the distance from cam surface 18 to axis A varies continuously along a spiral path of the cam from a maximum distance R₁ (about .177 inch - 0.44958 cm) near end 28 (Fig. 5) to a minimum distance R₂ (about .107 inch - 0.27178 cm) at end 30 (Fig. 5C).

The conformable interior of the resilient tubing provides a seal about an object, such as a guidewire, if present in the through-passage when the device is in the closed position.

Further, in the embodiment described in the figures above, the use of a freely rotating spherical compression member or ball allows for through-passage opening adjustments by slight rotation in either direction.

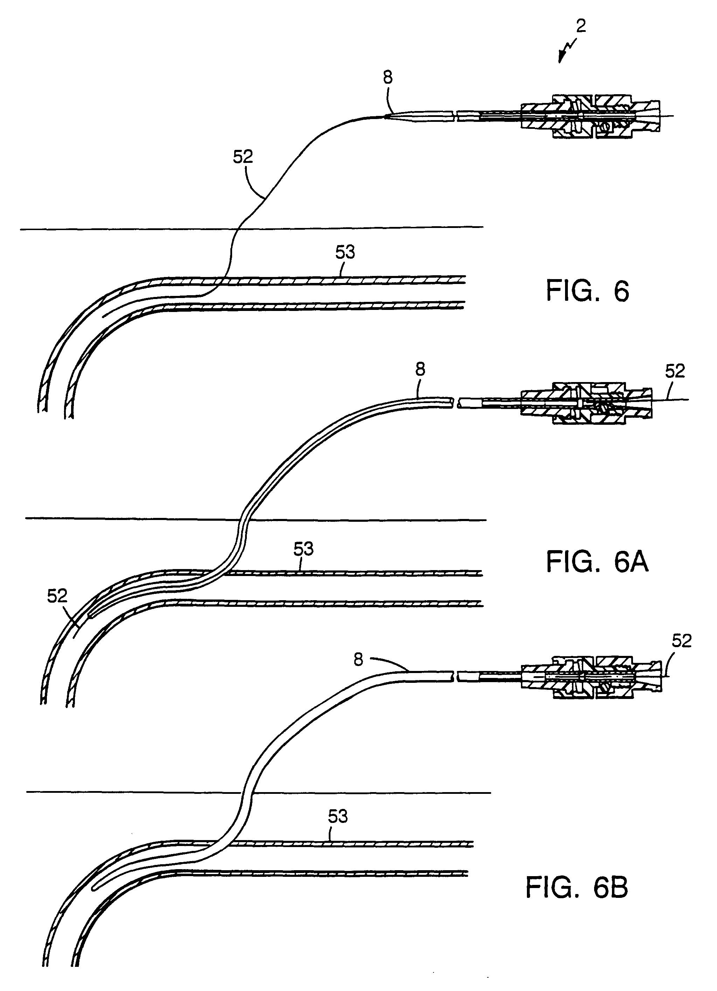

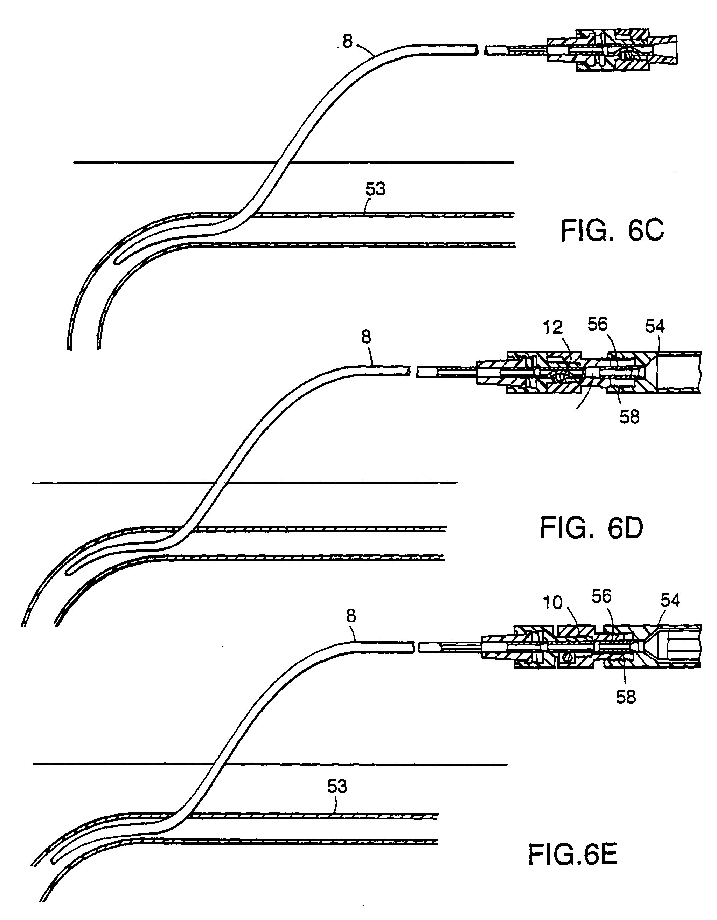

One particular use of the closure device is in the medical field for providing through-channel access to a catheter, such as an angiographic catheter. Referring to Fig. 6, in a typical catheterization operation such as an angiographic procedure, a guidewire 52 is positioned at the location of a desired arterial passageway (Fig. 6), such as the femoral artery for example, using an introducer needle (not shown) applying the Seldinger technique. An angiographic catheter 8 having attached thereto the closure device 2, is threaded over the guidewire 52 such that the catheter is positioned in the artery 53 (Fig. 6A). For this operation the closure device is in the open position, or alternatively, in a partially opened position to minimize backflow of blood alongside of the exterior of the catheter from the artery. The guidewire and catheter may be further positioned within the artery by manually rotating the proximal body member to the closed position, while gripping the guidewire 52 (Fig. 6A). The guidewire, catheter and device can thus be moved and torqued as a single unit to aid positioning as well as avoid punctures and other disturbances of the vessel (arterial passageway). With the catheter properly positioned, the device may be placed in the open position, again by manual rotation, to open the through-passage and allow removal of the guidewire (Fig. 6B). After removal of the guidewire, the valve is manually closed (Fig. 6C).

In the embodiment of Figs. 6 through 6E, the closure device can have the catheter permanently attached, as by insert molding, or removably attached as by matching luer connectors, to the distal end of the distal body member.

During the catheterization procedure, it is often desirable to inject fluids, such as contrast fluids for radiographic imaging, through the catheter. To effect injection, a syringe 54 with a distal end 56 adapted as a male luer lock fitting having locking thread 58 (end thread) is connected to the female luer adaptor 14 defined at the proximal end of the proximal body portion 12 by clockwise, "screw-on" rotation of syringe 54. The syringe rotates relative to the proximal end 12 before reaching a pre-engagement position (Fig. 6D). After reaching this position, at which point locking thread 58 is initially engaged, further rotation of the syringe causes simultaneous rotation of proximal body member 12 thereby automatically effecting opening of the through-passage (Fig. 6E). When the compression ball reaches the end of the cam surface and engages butt surface 46, rotation of the distal body portion is stopped and further rotation of the syringe seats the luer lock. Injection of fluid can then be commenced. After injection, rotation of the syringe in the opposite, counter-clockwise direction causes simultaneous rotation of proximal body member 12 of the device, thereby automatically closing the through-passage. After the through-passage is completely closed, further rotation of the proximal portion is prevented by butting of the body portions together, as discussed above, and the syringe may then overcome the resistance of the luer lock and rotate relative to the proximal portion and be unlocked and removed.

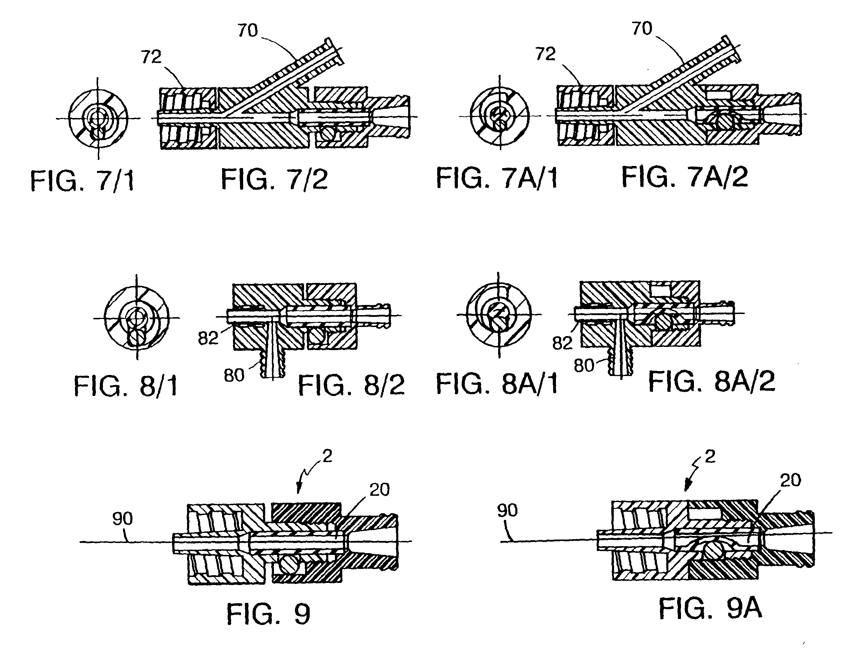

Referring to Figs. 7 and 7a, another embodiment of the device is shown to include a side arm 70 through which fluids may be injected, even after closure of the through-passage (Fig. 7a). Such a side arm may be provided on the distal portion of the device and include luer threads for connection to a catheter. This embodiment would be particularly useful in contrast injection after a guidewire passing through the closure device is locked into place.

Referring to Figs. 8 and 8a, another embodiment employing a side arm 80 at a right angle to the through-passage is illustrated, in which a male locking luer 82 is constructed integrally therewith.

Referring to Figs. 9 and 9a, the closure device 2 is shown for use as a quick hub attachment, by closing the channel 20 upon, for example, an elongated member. The elongated member 90 may be, for example, a small diameter guidewire, as shown, or a tube, and closure device 2 provides a convenient handle for the member as it is torqued during positioning in the body.

With the syringe or any other attachment to the proximal body member of the closure device removed, the device functions as a stop cock (though requiring counter clockwise rotation to close) for controlling flow through a tubing or other such elongated member 90, as shown in Figs. 9 and 9A, thereby replacing conventional stop cocks and for which no additional drawing is necessary.

Where the automatic opening feature upon connection of a device is not to be employed, threads 13 and 13' may be changed to right hand threads to enable this through-passage stop cock and gripper to close with conventional right hand rotation.

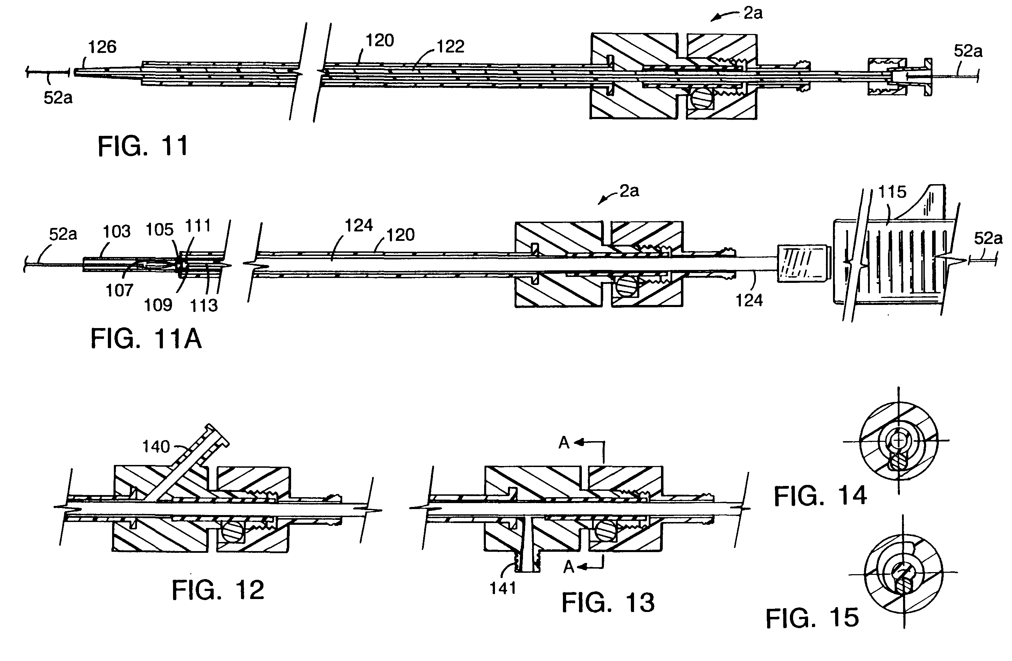

In a very important further embodiment, referring to Fig. 11, a closure device 2a is provided of much larger dimension, (device 2a preferably having an outer diameter of about 1 inch (2.54 cm), the tubing having an outer diameter of about 0.316 inch (0.80264 cm), and the through-passage having an inner diameter of about 0.170 inch - 0.4318 cm) and having the direction of its spiral cam surface and the direction of the threads between the parts of the housing extending in the opposite direction (right handed). This device is connected, e.g. by insert moulding, to a thin-walled sheath 120 of between 50 and 150 cm length to provide a catheter introducer sheath assembly, constructed to enable introduction of a placement catheter 124 for the placement of an element, such as a vena cava filter (one type of which is known as a Greenfield filter) into the body. (The closure device and sheath could be joined instead by any other means, e.g. matching luer connectors between the closure device and the sheath). The use of a vena cava filter for filtering out blood clots by its placement in the inferior vena cava is well-known in the medical field. See, e.g. U.S. Patent No. 3,952,747 to Kimmell, and U.S. Patent No. 4,817,600 to Herms et al. The general procedure followed in the embodiment to be illustrated is to use a guidewire (the guidewire may be routed into the desired position using the closure device of Fig. 2 as a gripping means).

Where a guidewire is used, the guidewire is routed through the vasculature in a manner where it can be watched fluoroscopically and manipulated accordingly. Thereafter the thin-walled sheath 120 with attached closure device 2a and containing an elongated flexible dilator 122, with exposed tip 126 (see figure 11) is introduced over the guidewire, and once placed, the dilator is withdrawn. Closing the valve of the closure device 2 forms an air-tight and fluid-tight seal around the guidewire, thereby preventing, for example, blood from flowing backwards through the sheath 122. Thus, the guidewire can be temporarily left in place inside the long flexible sheath when used with the closure device of the invention; reliable closure is established around the guidewire by an approximate one-half turn of the proximal body member relative to the distal body member by simple manual rotation of the proximal body portion by grip of the outer cylindrical surface.

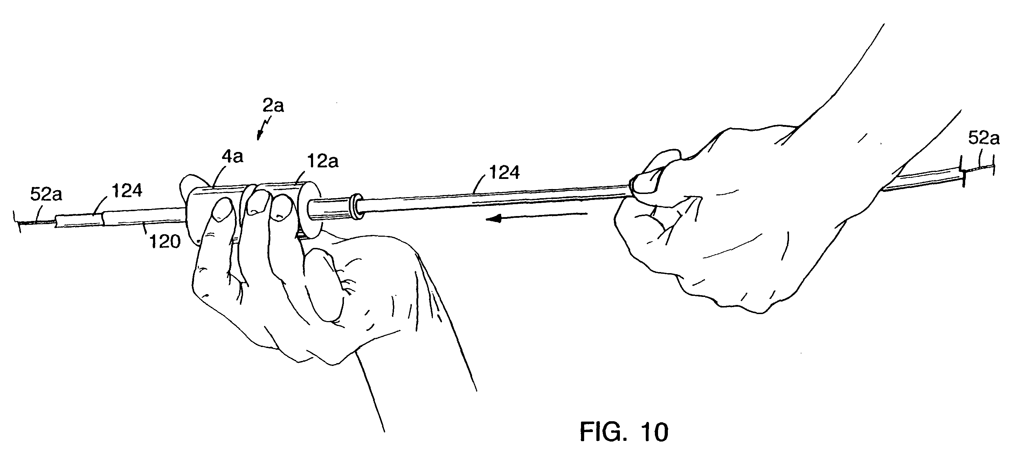

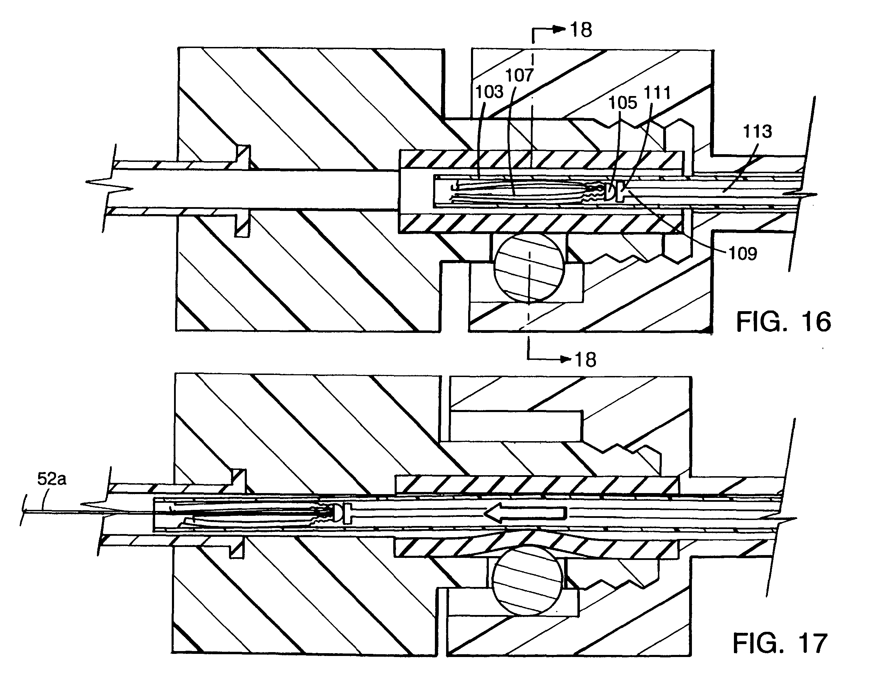

The placement catheter 124 containing the vena cava filter 105 is then introduced via the through-passage of the closure device 2a as shown in Fig. 10. For this purpose the proximal portion 12 of the closure device 2a is rotated to open position (in this case, using right hand threads, opening direction is counter clockwise.) After threading of the catheter 122 through the closure device 2a and sheath 120 has begun, as depicted in Fig. 10, the user manipulates the proximal body portion to slightly snug the elastomeric tube against the exterior of the catheter, sufficiently to reduce blood loss along the catheter but insufficient to impede desired sliding of the blood-lubricated catheter into the sheath, see Fig. 17.

The end portion of this catheter which first passes through the closure device is a metal sleeve 103 (Fig. 11a), within which is positioned the vena cava filter 105, characterized in part by its springy legs 107. This is delivered to the vena cava of the patient first by sliding the catheter through the closure device and the sheath and then drawing the sleeve proximally, releasing the filter from the end of the catheter. The use of a metal sleeve as the distal end portion of the catheter enclosing the filter facilitates release of the filter, which has outwardly springable legs, as shown from inside the catheter when the catheter is in position for final placement of the filter in the inferior vena cava.

The filter freely rests in the described metal sleeve of the catheter with a stabilizer 109 within the catheter 122 having a flat surface 111 immediately behind the filter. The stabilizer is connected to a tube 113, a sufficiently axially stiff structure to accomplish the stabilizing effect on the filter. When the catheter is positioned at the desired point in the vessel, the catheter 124 and its distal metal sleeve 103 is pulled proximally, while the stabilizer 109 remains stationary, by means of a reverse action trigger 115 attached to the proximal end of the proximal body member of the closure device. This reverse action operates so that pull-back on the trigger by the operator causes release of the filter from the catheter.

In the catheter placement embodiment of the invention, as mentioned above, the threads by which the proximal portion of the distal body member is connected to the distal portion of the proximal body member are right handed (reversed from the left-handed threading used in the syringe attachment embodiment of the invention). Thus in the catheter placement embodiment, the proximal body member is turned to the left (counter clockwise) relative to the distal body member to open the resilient silicone tubing. This open position is used for introducing the sheath and closure device over the guidewire which passes through the closure device and for introducing the catheter and contained vena cava filter into the sheath. The open (or partially open) position is also maintained during activation of a reverse action trigger at the proximal end of the catheter for deployment of the vena cava filter from the metal sleeve end of the catheter.

As shown in Fig. 12, this embodiment includes side arm 140 through which fluids may be injected after closure of the through-passage by the ball member, after removal of the dilator and prior to insertion of the catheter. This side arm is located on the distal side of the device and includes luer threads for connection to a supply tube that communicates with an elevated source or a to a syringe. This embodiment is useful for flushing the space within the catheter introducer sheath with saline solution containing heparin, to prevent blood clotting which could block the through-passage and obstruct later insertion of the catheter containing the vena cava filter.

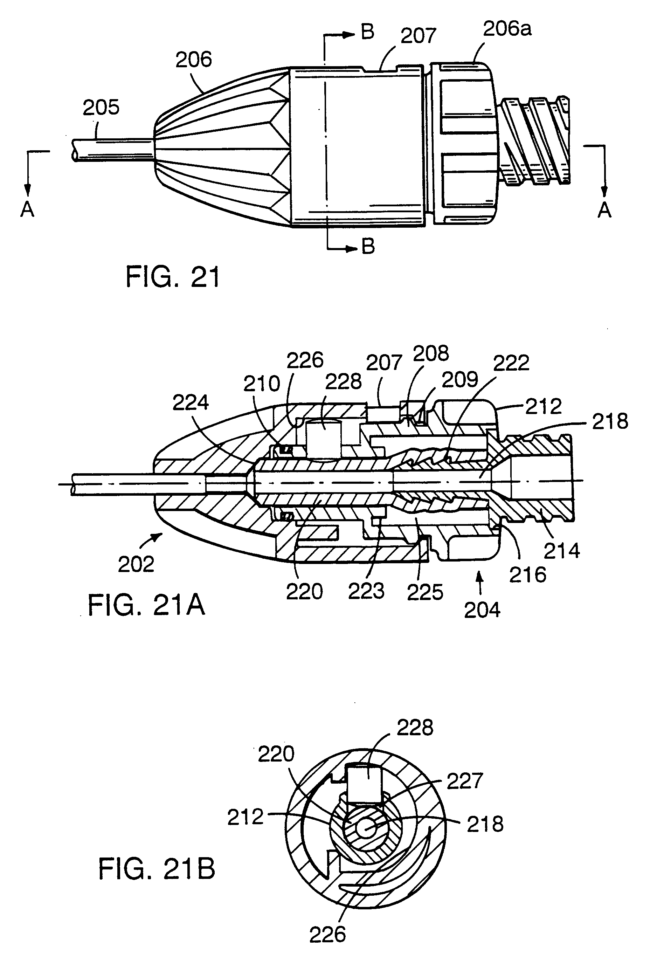

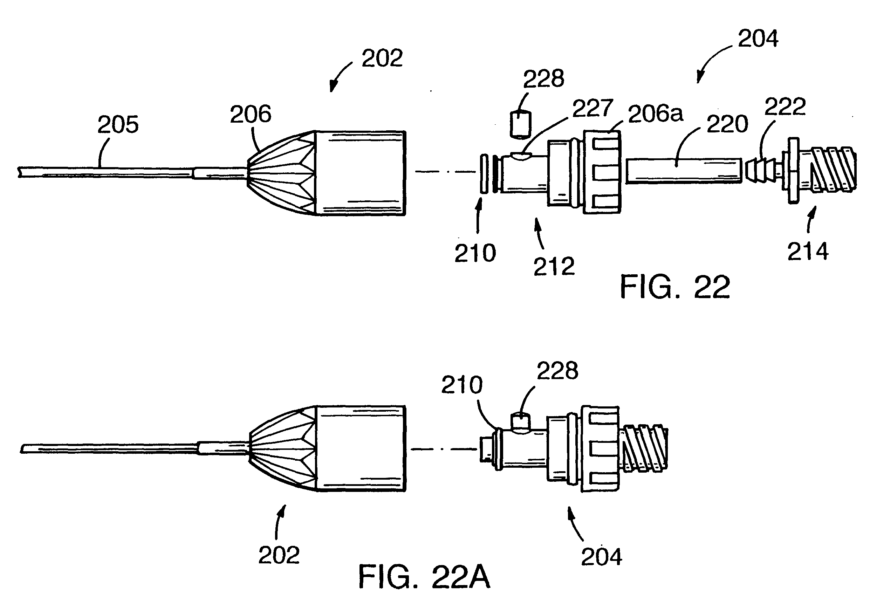

Referring to Figs. 21-21B, 22 and 22A a device is shown which is generally preferred for use in applications in which the valve will be subjected to a wide range of pressures, including high fluid pressure. The device includes a distal body member 202 and a proximal body member assembly 204, each having a gripping surface 206, 206a to facilitate relative rotation by the user. A catheter 205 extends from the distal body member. A window 207 in the distal body member enables the user to view indicia decribing the condition of the valve, e.g., "open" and "closed", on the surface of the underlying proximal body member assembly.

Distal body member 202 securely engages proximal body member assembly 204 by a snap fit engagement of circumferential ridge 208 on proximal body member assembly 204 with shoulder 209 on distal body member 202 (Fig. 21A). A sealing member 210, typically an elastomeric o-ring, disposed near the distal end of the proximal body member, ensures a reliable fluid-tight and air-tight seal between the distal and proximal body members. Should negative pressure be applied to the central lumen (e.g., by drawing back on a syringe connected to the proximal end of the proximal portion), the sealing member 210 provides a primary seal inhibiting air from entering the fluid path when the compression member is in the open position. Under pressurized conditions, the sealing member provides a back-up seal (primary sealing is provided by expansion of tubing member 220). As tubing member 220 will expand less under low pressure conditions, it is under low pressure that the sealing member is most important as a back-up.

Proximal body member assembly 204 includes a first part 212 and a second part 214, which may be pressure fit or bonded together at region 216 (Fig. 21A), e.g., by solvent or ultrasonic bonding. It is preferred, for optimal safety and reliability, that the two parts be bonded together.

A passage 218 extends along the longitudinal axis of the device, and is defined by the inner walls of the proximal and distal body members. A resilient tubing member 220 is disposed within and supported by the passage. The proximal end of the tubing is in sealing engagement with the second part 214, sealed for high pressure by the pressure fit of barbed fitting 222 within the resilient tubing. Sealing is aided by supporting member 223 which limits radial expansion of tubing member 220 and provides a back-up seal of the exterior of the tubing member to the supporting member under pressurized conditions. This back-up seal, in combination with the seal formed at region 216, provides a chamber 225 which will retain any fluid leakage. The distal end of tubing member 220 abuts a tapered surface 224 of distal body member 202.

Distal body member 202 includes an inner circumferential camming surface 226. A radial aperture 227 extends from the outer surface of part 212 to passage 218, and receives and retains a compression member 228. Rotation of distal body member 202 will thus cause the radial position of compression member 228, and thus the compression of the tubing member, to be adjusted, opening and closing the valve.

The material of resilient tubing member 220 is selected to enable it to seal upon itself, when collapsed by compression member 228, or seal upon a medical device such as a catheter or guidewire when present in passage 218.

Referring to Fig. 22, the device is assembled by first assembling proximal body member assembly 204. Barbed fitting 222 is inserted into the proximal end of tubing member 220, and the distal end of tubing member 220 inserted into part 212. Compression member 228 is placed in aperture 227, and sealing member 210 is fitted onto the distal end of part 212. Parts 212 and 214 are bonded together at region 216, forming the proximal body member assembly shown in Fig. 22A. The proximal body member assembly is then snap fit into the distal body member, forming the complete device shown in Fig. 21.

It will be apparent that our closure devices can be embodied in many forms too numerous to attempt to mention. As examples only, the distal body member of the closure device could carry an integral, deflectable hinged member in place of the separate spherical compression element as described, as a valve component to be disposed between the cam surface of the proximal body member and the resilient tube to effectuate opening and closing of the tube. Also, although only one cam and associated compression member has been described, the closure device is not so limited. For example, two cams could be used with two compression balls, hinged legs, cantilevered elements, or the like on opposing sides, to carry out the tube-opening and closing by means of displacement of the elements in the closure device by co-rotation of respective cam surfaces. Thus, although a ball-form or sphere has been described as the compression member in the device, the member does not necessarily have to be of that form, nor does it necessarily need to be limited to only one element that bears radially inward on the tubing. Indeed a resilient wall portion of an otherwise rigidly defined passage that can be deflected under the influence of the compression device to close the passage can be employed in place of the resilient tube.

Also, the embodiments described use either right-handed or left-handed threads to join the distal and proximal body members of the closure device and cause slight axial movement as the compression member moves in opening or closing the tube. The means of joinder and movement between the distal and proximal body members could also be found in other rotatable connections to give the same type of operation, some in which there is no axial advance at all, so that the closure device is not limited to threaded surfaces for joining the component members.

For the syringe-actuated arrangement, to obtain some of the advantages, it is possible to employ other two-part rotary valve constructions, such as two relatively rotatable disks each having an aperature which through rotation of the disks line up to define the passage and through opposite rotation move out of alignment to close the passage.

## Claims

1. A closure device (2) to which a cooperative component (8) is attachable for communication therebetween, said closure device comprising a rotatable body portion (4) including a connector (6) for connection to and disconnection from a mating connector (7) of the cooperative component (8) by relative rotation between said rotatable body portion (4) and the cooperative component (8), and a relatively stationary body portion (12) of said closure device, said rotatable body portion (4) being rotatable relative to said stationary body portion (12) between an open position in which a passage (20) is defined through said closure device and a closed position in which said passage is closed; the closure device being **characterised in that** said body portions are constructed and arranged such that the direction of relative rotation of said connectors for connection of said rotatable body portion (4) to said cooperative component (8) corresponds to the direction of rotation of said rotatable portion (4) relative to said stationary body portion (12) toward said open position to also open said passage (20) and the direction of relative rotation of said connectors that disconnect said rotatable body portion (4) from said cooperative component (8) corresponds to the direction of rotation of said rotatable body portion (4) relative to said stationary body portion (12) toward said closed position to also close said passage (20).

2. A closure device according to Claim 1, **characterized in** further comprising a resilient member (22), preferably a resilient section of tubing, having an axis at least partially defining said passage (20); **in that** said rotatable body portion (4) includes an internal cam (18) having a cam surface oriented about and spaced from the axis of said passage (20), said cam having a first surface portion (30) disposed relatively closer to said axis than a circumferentially spaced second surface portion (28); and **in that** a radially movable compression member (24) is disposed between and in such contact with said cam surface and the exterior of said resilient member (22) that said rotation for connection said component (8) to said closure device (2) causes said cam surface to adjust the radial compression of said resilient member (22) further from said axis to open said passage (20), and said rotation for disconnecting said component (8) from said closure device causes said cam surface to adjust the radial compression of said resilient member (22) to said axis to close said passage (20).

3. A closure device according to Claim 2, further **characterized in that** said relatively stationary body portion (12) fixes the position of said tubing (22).

4. A closure device according to Claim 2, further **characterized in that** rotational force required to rotate said rotatable body portion (4) to effect change in the compression of said resilient member (22) to open and close said passage (20) is less than the rotational force needed, respectively, to complete the connection and disconnection of said cooperative component (8) relative to said device (2).

5. A closure device according to Claim 4, further **characterized in that** said rotatable body portion (4) is threadably attached about said axis to said relatively stationary body portion (12), the threads (13, 13') thereof being opposite to the direction of rotation that enables said device (2) to be rotatably connected to said cooperative component (8), whereby rotation to connect said cooperative component (8) to said device (2) causes said body portions to move apart.

6. A closure device according to Claim 5, further **characterized in that** a stop surface (46) is defined at an end of said second cam surface portion (28), said stop surface (46) being arranged to engage said compression member (24) and stop relative motion apart of said body portions (4, 12) when said compression member (24) reaches passage-opening position preventing disassembly of said body portion by blocking further rotation and said rotatable and relatively stationary body portions are constructed to engage each other and stop threaded-together motion when said compression member reaches a passage-closing position.

7. A closure device according to Claims 2 or 4, further **characterized in that** said closure (2) device is adapted for use as a medical closure device, said passage (20) being arranged to pass a pressurized fluid or a medical device into or out of a living body and the proximal end (14) of said device being constructed to be connected to a cooperative catheter or a syringe.

8. A closure device according to Claim 7, further **characterized in that** the distal end (6) of said closure device is constructed to be connected to a catheter, preferably the catheter being integrally moulded with the said distal end.

9. A closure device according to Claim 2, further **characterized in that** said first surface portion (30) of said cam (18) positions said compression member (24), preferably a single ball-form member, radially to completely close in fluid-tight manner said through-passage (20) and said second surface portion (28) positions said compression member to fully open said through-passage (20), said cam (18) preferably defining a smooth spiral-form surface.

10. A device according to Claim 6, **characterised in** further comprising a screw thread on said rotatable body portion (4), said screw thread being configured to engage a screw thread on said cooperative component (8), and **in that** said cam (18) defines a spiral-form surface with an abrupt transition from the end of said second cam surface portion (28), furthest from the axis, to a portion of said rotatable body portion (4) closer to the axis in a manner interacting with said compression member (24), said manner of interacting permitting continual rotation during assembly of said rotatable body portion (4) in a screw-on direction in which the compression member (24) is gradually progressively compressed against said resilient member (22) to the maximum at which state it remains until further rotation causes the second portion (28) of the cam surface to reach said compression member (24) permitting said compression member to spring outwardly to permit continued rotation of said rotatable body portion (4), whereas opposite rotation of said rotatable body portion (4) along said threads is limited by stopping of said compression member (24) against said abrupt transition of said rotatable body portion.

11. A closure device according to Claim 6, further **characterized in that** said second stop (46) blocks further axial advance along said thread of said rotatable body portion (4).

12. A closure device according to Claim 11, further **characterized in that** said relatively stationary body portion (12) fixes the position of said resilient member (22) and defines a radially extending aperture (26) in which said compression member (24) is axially confined, and permitted to move radially against said resilient member (22).

13. A closure device according to Claim 2, further **characterized in that** said tubing (22) is compressed axially and confined radially for sealing engagement of surfaces (42,38) at its proximal and distal ends.

14. A closure device according to Claim 2, further **characterized in that** said body portions and said passage (20) are sized for passage therethrough of a device and provide, when said closure device (2) is closed upon said device, means for gripping and torquing said device.

15. A closure device according to Claim 2, further **characterized in that** said body is attachable at its proximal and distal ends to tubing members for controlling the flow of fluid therethrough.

16. A closure device according to Claim 2, further **characterized in that** said rotatable body portion (4) has an external surface exposed for engagement and rotatable operation by the hand of a user.

17. A closure device according to Claim 2, further **characterized in that** said rotation of said rotatable body portion (4) to open and close said through-passage is substantially one-half turn.

18. A closure device according to Claim 1, further **characterized in that** said body portions, in open position, define a through-passage through which a device can pass.

19. A closure device according to Claim 18, further **characterized in that** the wall defining said passage (20) is constructed to close and seal or grip a device inserted into said through-passage.

20. A closure device according to Claim 7, **characterized in** further including a flexible introducer sheath (120) joined to the distal end of said closure device, and a catheter (124) containing a vena-cava filter (105) and a stabilizer (109) for placement of said filter, said introducer sheath (120) and said closure device (2a) defining a passage, preferably of the order of 1/8 inch (0.3175cm) sized to pass said catheter (124) therethrough, said flexible introducer sheath (120) and said closure device (2a) comprising a catheter introducer sheath assembly, and said catheter (124) and said catheter introducer sheath assembly being contained in a catheter introducer kit.

21. A closure device according to Claim 20, further **characterized in that** said rotatable body portion (4) is threadably attached about said axis to said relatively stationary body portion (12) and said compression member (24) is adjustable by manual manipulation of said rotatable body portion (4) to selected intermediate rotatable positions relative to said relatively stationary body portion (12) to cause said resilient member (22) to lightly engage the sides of said catheter (124) being inserted through said catheter introducer sheath assembly to impede outward blood flow along the sides of said catheter while permitting slidable insertion thereof.

22. A closure device according to Claim 20, further **characterized in** having a removable elongate dilator (122) disposed in said flexible introducer sheath (120), said dilator (122) having a tapered distal portion (126) extending distally beyond the flexible introducer sheath.

23. A closure device according to Claim 23, further **characterized in that** said dilator (122) has a small axial passage enabling said dilator (122), flexible introducer sheath (120) and closure device (2a) to be slid over a pre-placed guidewire for guiding the sheath (120) into a predetermined position.

24. A medical closure device for providing a passage (20) into or out of a medical cooperative component (8) the medical cooperative component (8) being adapted to be inserted into a human or animal body, the medical closure device providing passage for both pressurized fluids and a medical device, said medical closure device being **characterized in** comprising: a closure device according to Claim 1, the connector (6) of the rotatable body portion (4) of which is adapted for connection to and disconnection from a mating connector (7) of said medical cooperative component (8).

25. A medical closure device according to Claim 24, **characterized in** further comprising: a resilient member (22) at least partially defining said passage (20), said passage having an axis and being arranged for passage therethrough of a selected medical device and pressurized fluid, said resilient member (22) being of material selected, in response to radial pressure applied to said resilient member (22), to form a pressure seal upon said medical device when said medical device is present in said passage (20) and upon an opposing surface defining said passage when said medical device is not present; a proximal body portion (12) including first and second members, said first member having a pressure fitting in sealing engagement with and mounting said resilient member (22) and said second member being disposed about and supporting said resilient member (22); a distal body portion (4) including an internal cam (18) having a cam surface oriented about and spaced from the axis of said passage (20) with a first surface portion (30) disposed relatively closer said axis than a circumferentially spaced second surface portion (28); and a compression member (24) positioned in a radially extending aperture (26) in said proximal body portion (12) and biased radially outwardly by said resilient member (22) to maintain contact with said cam surface; and being further **characterized in that** said proximal and distal body portions (4, 12) are connected together in a manner permitting their relative rotation about said axis, said body portions (4, 12) and said resilient member (22) being sealed together in a manner to withstand, without leakage, fluid under pressure in said passage; and **in that** said connected body portions (4, 12) are cooperatively related, whereby, upon relative rotation of said body portions (4, 12), said cam surface is moved relative to said compression member (24) to cause radial displacement of said compression member (24) closer to the axis of said passage to cause said resilient member (22) to grip and pressure seal upon the exterior of said medical device, when present in said passage, or upon said opposing surface defining said passage when said medical device is not present.

26. A closure device according to Claim 14, further **characterized in that** said device further comprises a sealing member (210) mounted on said second part of said proximal body portion (12) to enhance sealing engagement between said distal and said proximal body portions (4, 12).

27. A method of opening or closing a passage through which both pressurized fluids and a medical device can pass into or out of the body, comprising the steps of: connecting or disconnecting a said cooperative component (8) relative to said rotatable body portion (4) of a closure device according to Claim 1 to open or close said passage, respectively, by relative rotation between said rotatable body portion (4) and the cooperative component (8).

28. A method according to Claim 27, further comprising the steps of: introducing a medical device through said passage; and while said device is within said passage, causing relative rotation of said second body portion in the direction that disconnects said component from said closure device to cause a resilient member (22) within said closure device to grip and pressure seal upon the exterior of said device.

29. A method according to Claim 27, wherein said cooperative component is a syringe for supplying pressurized fluids.

## Patentansprüche

1. Verschlusseinrichtung (2), an die eine kooperative Komponente (8) befestigt werden kann, um mit dieser zu kommunizieren, wobei diese Verschlusseinrichtung gebildet wird aus einem drehbaren Gehäuseabschnitt (4) mit einem Verbindungsstück (6) zur Verbindung mit und zur Trennung von einem passenden Verbindungsstück (7) der kooperativen Komponente (8) durch eine relative Drehbewegung zwischen dem drehbaren Gehäuseabschnitt (4) und der kooperativen Komponente (8) und aus einem relativ stationären Gehäuseabschnitt (12) der Verschlusseinrichtung, wobei der drehbare Gehäuseabschnitt (4) gegenüber dem relativ stationären Gehäuseabschnitt (12) drehbar ist zwischen einer offenen Position, in der eine Passage (20) durch die Verschlusseinrichtung definiert wird, und einer geschlossenen Position, in welcher die Passage geschlossen ist; **dadurch gekennzeichnet, dass** die Gehäuseabschnitte so aufgebaut und angeordnet sind, dass die Richtung der relativen Drehung der Verbindungsstücke zum Verbinden des drehbaren Gehäuseabschnittes (4) mit der kooperativen Komponente (8) mit der Drehrichtung des drehbaren Gehäuseabschnitts (4) im Verhältnis zum stationären Gehäuseabschnitt (12) in Richtung der offenen Position zum gleichzeitigen Öffnen der Passage (20) übereinstimmt und dass die Richtung der relativen Drehung der Verbindungsstücke zum Trennen des drehbaren Gehäuseabschnittes (4) von der kooperativen Komponente (8) mit der Drehrichtung des drehbaren Gehäuseabschnitts (4) im Verhältnis zum stationären Gehäuseabschnitt (12) in Richtung der geschlossenen Position zum gleichzeitigen Schließen der Passage (20) übereinstimmt.

2. Verschlusseinrichtung entsprechend Anspruch 1, **dadurch gekennzeichnet, dass** sie des Weiteren gebildet wird aus einem elastischen Element (22) - bei dem es sich bevorzugterweise um einen elastischen Abschnitt einer Rohrleitung handelt -, das eine Achse aufweist, die zumindest einen Teil der Passage (20) definiert; dass der drehbare Gehäuseabschnitt (4) einen internen Nocken (18) hat, dessen Nockenoberfläche in einem Abstand um die Achse der Passage (20) verläuft, wobei der Nocken einen ersten Oberflächenabschnitt (30) aufweist, der sich relativ betrachtet näher an der Achse befindet als ein mit Abstand zum Umfang der Achse angeordneter zweiter Oberflächenabschnitt (28); und dass sich zwischen und im Kontakt mit der Nockenoberfläche und dem Äußeren des elastischen Elements (22) ein radial bewegliches Kompressionselement (24) befindet, so dass die Drehung zum Verbinden der Komponente (8) mit der Verschlusseinrichtung (2) in Folge der Nockenoberfläche eine solche Anpassung der radialen Kompression des elastischen Elements (22) von der Achse weg bewirkt, dass sich die Passage (20) öffnet, und so dass die Drehung zum Trennen der Komponente (8) von der Verschlusseinrichtung in Folge der Nockenoberfläche eine solche Anpassung der Kompression des elastischen Elements (22) auf die Achse zu bewirkt, dass die Passage (20) geschlossen wird.

3. Verschlusseinrichtung entsprechend Anspruch 2, des Weiteren **dadurch gekennzeichnet, dass** der relativ stationäre Gehäuseabschnitt (12) eine Fixierung der Position der Rohrleitung (22) bewirkt.

4. Verschlusseinrichtung entsprechend Anspruch 2, des Weiteren **dadurch gekennzeichnet, dass** die Rotationskraft, die zur Drehung des drehbaren Gehäuseabschnitts (4) mit dem Ziel der Änderung der Kompression des elastischen Elements (22) zum Öffnen und Schließen der Passage (20) erforderlich ist, geringer als die Rotationskraft ist, die jeweils zum Herstellen der Verbindung der kooperativen Komponente (8) mit der Einrichtung (2) bzw. zum Trennen der kooperativen Komponente (8) von der Einrichtung (2) erforderlich ist.

5. Verschlusseinrichtung entsprechend Anspruch 4, des Weiteren **dadurch gekennzeichnet, dass** der drehbare Gehäuseabschnitt (4) um seine Achse schraubbar am relativ stationären Gehäuseabschnitt (12) befestigt wird, wobei dessen Gewinde (13, 13') entgegengesetzt zur Drehrichtung verlaufen, welche die drehbare Verbindung der Einrichtung (2) mit der kooperativen Komponente (8) ermöglicht, wodurch die Drehung zum Verbinden der kooperativen Komponente (8) mit der Einrichtung (2) dazu führt, dass die Gehäuseabschnitte voneinander getrennt werden.

6. Verschlusseinrichtung entsprechend Anspruch 5, des Weiteren **dadurch gekennzeichnet, dass** sich an einem Ende des zweiten Nockenoberflächenabschnitts (28) eine Anschlagfläche (46) befindet, wobei diese Anschlagfläche (46) so angeordnet ist, dass sie in das Kompressionselement (24) eingreift und die relative Bewegung von den Gehäuseabschnitten (4, 12) weg stoppt, wenn das Kompressionselement (24) die Position erreicht, in der die Passage (20) geöffnet ist, wodurch die Demontage des Gehäuseabschnitts verhindert wird, indem die weitere Drehung gestoppt wird; und der drehbare und der relativ stationäre Gehäuseabschnitt sind so konstruiert, dass sie ineinander eingreifen und die Zusammenschraubbewegung stoppen, wenn das Kompressionselement die Position erreicht, in der die Passage (20) geschlossen ist.

7. Verschlusseinrichtung entsprechend den Ansprüchen 2 oder 4, des Weiteren **dadurch gekennzeichnet, dass** die Verschlusseinrichtung (2) für die Verwendung als medizinische Verschlusseinrichtung angepasst ist, wobei die Passage (20) so angeordnet ist, dass sie eine unter Druck stehende Flüssigkeit oder ein medizinisches Gerät in einen lebenden Körper hineinleiten oder aus diesem herausleiten kann, und das proximale Ende (14) der Einrichtung so konstruiert ist, dass es mit einem kooperativen Katheter oder einer Injektionsspritze verbunden werden kann.

8. Verschlusseinrichtung entsprechend Anspruch 7, des Weiteren **dadurch gekennzeichnet, dass** das distale Ende (6) der Verschlusseinrichtung so konstruiert ist, dass es mit einem Katheter verbunden werden kann, wobei es sich bevorzugterweise um den Katheter handelt, der integral in das distale Ende eingegossen ist.

9. Verschlusseinrichtung entsprechend Anspruch 2, des Weiteren **dadurch gekennzeichnet, dass** der erste Oberflächenabschnitt (30) des Nockens (18) das Kompressionselement (24), bei dem es sich bevorzugterweise um ein aus einer Kugel bestehendes Element handelt, radial in eine solche Position bringt, dass die durchgehende Passage (20) vollständig flüssigkeitsdicht abgeschlossen wird, und dass der zweite Oberflächenabschnitt (28) das Kompressionselement in eine solche Position bringt, dass die durchgehende Passage (20) vollständig geöffnet ist, wobei der Nocken (18) bevorzugterweise eine gleichmäßig verlaufende spiralförmige Oberfläche aufweist.

10. Verschlusseinrichtung entsprechend Anspruch 6, **dadurch gekennzeichnet, dass** sie des Weiteren ein Schraubengewinde auf dem drehbaren Gehäuseabschnitt (4) aufweist, wobei dieses Schraubengewinde so konfiguriert ist, dass es in ein Schraubengewinde auf der kooperativen Komponente (8) eingreift, und dadurch dass der Nocken (18) eine spiralförmige Oberfläche aufweist mit einem abrupten Übergang vom Ende des ersten Nockenoberflächenabschnitts (28) - der sich am weitesten von der Achse entfernt befindet - zu einem näher an der Achse gelegenen Abschnitt des drehbaren Gehäuseabschnitts (4), so dass es zur Interaktion mit dem Kompressionselement (24) kommt, wobei diese Interaktion eine kontinuierliche Drehbewegung bei der Montage des drehbaren Gehäuseabschnitts (4) in Aufschraubrichtung ermöglicht, bei der das Komprimierungselement (24) allmählich zunehmend gegen das elastische Element (22) gepresst wird, und zwar bis zu einem Maximalstatus, in dem es verbleibt, bis eine weitere Drehung dafür sorgt, dass der zweite Abschnitt (28) der Nockenoberfläche das Komprimierungselement (24) erreicht, wodurch sich das Komprimierungselement nach außen bewegen kann, um eine dauerhafte Drehung des drehbaren Gehäuseabschnitts (4) zu ermöglichen, wohingegen die entgegengesetzte Drehung des drehbaren Gehäuseabschnitts (4) entlang der Gewinde dadurch eingeschränkt wird, dass das Komprimierungselement (24) durch den abrupten Übergang des drehbaren Gehäuseabschnitts gestoppt wird.

11. Verschlusseinrichtung entsprechend Anspruch 6, des Weiteren **dadurch gekennzeichnet, dass** die zweite Anschlagfläche (46) die weitere axiale Fortbewegung entlang des Gewindes des drehbaren Gehäuseabschnitts (4) blockiert.

12. Verschlusseinrichtung entsprechend Anspruch 11, des Weiteren **dadurch gekennzeichnet, dass** der relativ stationäre Gehäuseabschnitt (12) die Position des elastischen Elements (22) fixiert und eine radial verlaufende Öffnung (26) definiert, von der das Komprimierungselement (24) axial eingeschlossen wird, so dass dieses sich lediglich radial gegen das elastische Element (22) bewegen kann.

13. Verschlusseinrichtung entsprechend Anspruch 2, des Weiteren **dadurch gekennzeichnet, dass** die Rohrleitung (22) axial zusammengedrückt und radial fixiert wird, um einen Dichtungskontakt der Oberflächen (42, 38) an seinen proximalen und distalen Enden zu erzielen.

14. Verschlusseinrichtung entsprechend Anspruch 2, des Weiteren **dadurch gekennzeichnet, dass** die Gehäuseabschnitte und die Passage (20) so bemessen sind, dass durch sie ein Gerät geführt werden kann und dass sie beim Schließen der Verschlusseinrichtung (2) um dieses Gerät eine Vorrichtung zum Einspannen und Drehen dieses Geräts bilden.

15. Verschlusseinrichtung entsprechend Anspruch 2, des Weiteren **dadurch gekennzeichnet, dass** das Gehäuse an seinem proximalen und distalen Ende an Rohrleitungen angeschlossen werden kann, um den Durchfluss der Flüssigkeit zu steuern.

16. Verschlusseinrichtung entsprechend Anspruch 2, des Weiteren **dadurch gekennzeichnet, dass** der drehbare Gehäuseabschnitt (4) eine freiliegende Außenfläche aufweist, die durch die Hand eines Benutzers ergriffen und gedreht werden kann.

17. Verschlusseinrichtung entsprechend Anspruch 2, des Weiteren **dadurch gekennzeichnet, dass** die Drehung des drehbaren Gehäuseabschnitts (4) zum Öffnen und Schließen der durchgehenden Passage im Wesentlichen eine halbe Umdrehung umfasst.

18. Verschlusseinrichtung entsprechend Anspruch 1, des Weiteren **dadurch gekennzeichnet, dass** die Gehäuseabschnitte in der offenen Position eine durchgehende Passage bilden, durch welche ein Gerät geführt werden kann.

19. Verschlusseinrichtung entsprechend Anspruch 18, des Weiteren **dadurch gekennzeichnet, dass** die Wand, welche die Passage (20) definiert, so konstruiert ist, dass sie ein in die durchgehende Passage eingeführtes Gerät beim Verschließen abdichtet oder ergreift.

20. Verschlusseinrichtung entsprechend Anspruch 7, **dadurch gekennzeichnet, dass** sie des Weiteren besteht aus einer flexiblen Einführungshülle (120), die mit dem distalen Ende der Verschlusseinrichtung verbunden ist, und aus einem Katheter (124) mit einem Hohlvenenfilter (105) und einem Stabilisator (109) zur Platzierung des Filters, wobei die Einführungshülle (120) und die Verschlusseinrichtung (2a) eine Passage bilden, bevorzugterweise in der Größenordnung von 0,3175 cm (1/8 Zoll), durch die der Katheter (124) passieren kann, und wobei die flexible Einführungshülle (120) und die Verschlusseinrichtung (2a) eine Kathetereinführungshüllen-Baugruppe enthalten und der Katheter (124) und die Kathetereinführungshüllen-Baugruppe Bestandteil eines Kathetereinführungssatzes sind.

21. Verschlusseinrichtung entsprechend Anspruch 20, des Weiteren **dadurch gekennzeichnet, dass** der drehbare Gehäuseabschnitt (4) um seine Achse schraubbar am relativ stationären Gehäuseabschnitt (12) befestigt wird und dass das Komprimierungselement (24) durch manuelle Betätigung des drehbaren Gehäuseabschnitts (4) auf ausgewählte Zwischenstellungen der Drehung in Bezug auf den relativ stationären Gehäuseabschnitt (12) eingestellt werden kann, so dass das elastische Element (22) leicht in die Seiten des Katheters (124) eingreift, der durch die Kathetereinführungshüllen-Baugruppe eingeführt wird, um den nach außen gerichteten Blutfluss entlang den Seiten des Katheters zu verhindern und gleichzeitig die gleitende Einführung des Katheters zu ermöglichen.

22. Verschlusseinrichtung entsprechend Anspruch 20, des Weiteren **dadurch gekennzeichnet, dass** sie über einen herausnehmbaren langen und dünnen Dilatator (122) verfügt, der sich in der flexiblen Einführungshülle (120) befindet, wobei der Dilatator (122) einen spitz zulaufenden Distalabschnitt (126) hat, der sich hinter der flexiblen Einführungshülle in distaler Richtung fortsetzt.

23. Verschlusseinrichtung entsprechend Anspruch 22, des Weiteren **dadurch gekennzeichnet, dass** der Dilatator (122) eine kleine axiale Passage aufweist, durch die der Dilatator (122), die flexible Einführungshülle (120) und die Verschlusseinrichtung (2a) über einen vorher platzierten Führungsdraht geschoben werden können, der die Hülle (120) in eine vorbestimmte Position führt.

24. Medizinische Verschlusseinrichtung zur Bereitstellung einer Passage (20) in eine medizinische kooperative Komponente (8) bzw. aus dieser heraus, wobei die medizinische kooperative Komponente (8) zur Einführung in einen menschlichen oder tierischen Körper angepasst ist und die medizinische Verschlusseinrichtung eine Passage sowohl für unter Druck stehende Flüssigkeiten als auch für ein medizinisches Gerät bereitstellt und wobei die medizinische Verschlusseinrichtung **dadurch gekennzeichnet ist, dass** sie gebildet wird aus: einer Verschlusseinrichtung entsprechend Anspruch 1, dessen Verbindungsstück (6) des drehbaren Gehäuseabschnitts (4) zur Verbindung mit und zur Trennung von einem passenden Verbindungsstück (7) der medizinischen kooperativen Komponente (8) angepasst ist.

25. Medizinische Verschlusseinrichtung entsprechend Anspruch 24, **dadurch gekennzeichnet, dass** sie des Weiteren gebildet wird aus: einem elastischen Element (22), das zumindest zum Teil die Passage (20) definiert, wobei die Passage eine Achse aufweist und so angeordnet ist, dass durch sie ein ausgewähltes medizinisches Gerät und unter Druck stehende Flüssigkeit geführt werden kann, und wobei das elastische Element (22) aus einem Material besteht, das entsprechend dem auf das elastische Element (22) ausgeübten Radialdruck ausgewählt wird, um beim Vorhandensein des medizinischen Geräts in der Passage (20) eine Druckdichtung mit dem medizinischen Gerät zu bilden und um beim Nichtvorhandensein des medizinischen Geräts eine Druckdichtung mit einer gegenüberliegenden Fläche zu bilden, welche die Passage definiert; aus einem proximalen Gehäuseabschnitt (12) aus einem ersten und einem zweiten Element, wobei das erste Element ein Druckverbindungsstück zum abdichtenden Kontakt mit dem elastischen Element (22) und zur Befestigung desselben aufweist und das zweite Element um das elastische Element (22) angeordnet ist und dieses trägt; aus einem distalen Gehäuseabschnitt (4) mit einem internen Nocken (18), dessen Nockenoberfläche in einem Abstand um die Achse der Passage (20) verläuft und einen ersten Oberflächenabschnitt (30) hat, der sich relativ betrachtet näher an der Achse befindet als ein mit Abstand zum Umfang der Achse angeordneter zweiter Oberflächenabschnitt (28); und aus einem Kompressionselement (24), das in einer radial verlaufenden Öffnung (26) im proximalen Gehäuseabschnitt (12) positioniert ist und durch das elastische Element (22) radial nach außen vorgespannt wird, um den dauerhaften Kontakt zur Nockenoberfläche zu gewährleisten; und des Weiteren **dadurch gekennzeichnet, dass** die proximalen und distalen Gehäuseabschnitte (4, 12) in einer Weise miteinander verbunden sind, dass ihre relative Drehung um die Achse ermöglicht wird, wobei die Gehäuseabschnitte (4, 12) und das elastische Element (22) abdichtend miteinander verbunden sind, so dass unter Druck stehende Flüssigkeit ohne Leckverluste in der Passage festgehalten werden kann; und dass die verbundenen Gehäuseabschnitte (4, 12) kooperativ miteinander in Beziehung stehen, wodurch die Nockenoberfläche bei einer relativen Drehung der Gehäuseabschnitte (4, 12) relativ zum Kompressionselement (24) bewegt wird, was zu einer radialen Verschiebung des Kompressionselements (24) in Richtung der Achse der Passage führt, wodurch das elastische Element (22) auf die Außenseite des medizinischen Geräts gepresst wird und dieses druckdicht abdichtet, wenn das medizinische Gerät in der Passage vorhanden ist, oder auf die gegenüberliegende, die Passage definierende Fläche drückt und diese druckdicht abdichtet, wenn das medizinische Gerät nicht vorhanden ist.

26. Verschlusseinrichtung entsprechend Anspruch 14, des Weiteren **dadurch gekennzeichnet, dass** die Einrichtung außerdem gebildet wird aus einem Dichtungselement (210), das auf dem zweiten Teil des proximalen Gehäuseabschnitts (12) montiert ist, um den Dichtungskontakt der distalen und des proximalen Gehäuseabschnitte (4, 12) zu verbessern.

27. Verfahren zum Öffnen und Schließen einer Passage, durch welche sowohl unter Druck stehende Flüssigkeiten als auch ein medizinisches Gerät in den Körper eingeführt und aus dem Körper entfernt werden können, das folgende Schritte umfasst: Verbinden oder Trennen einer kooperativen Komponente (8) mit dem bzw. von dem drehbaren Gehäuseabschnitt (4) einer Verschlusseinrichtung entsprechend Anspruch 1 zum Öffnen bzw. Schließen der Passage durch eine relative Drehung zwischen dem drehbaren Gehäuseabschnitt (4) und der kooperativen Komponente (8).

28. Verfahren entsprechend Anspruch 27, das des Weiteren folgende Schritte umfasst: Einführen eines medizinischen Geräts durch die Passage; und - während das Gerät sich in der Passage befindet-Bewirken einer relativen Drehung des zweiten Gehäuseabschnitts in die Richtung, in der die Komponente von der Verschlusseinrichtung getrennt wird, um zu bewirken, dass ein elastisches Element (22) im Inneren der Verschlusseinrichtung die Außenseite des Geräts ergreift und druckdicht abdichtet.

29. Verfahren entsprechend Anspruch 27, bei dem es sich bei der kooperativen Komponente um eine Injektionsspritze für unter Druck stehende Flüssigkeiten handelt.

## Revendications

1. Dispositif de fermeture (2) auquel un composant coopérant (8) peut être fixé en vue d'une communication entre eux, ledit dispositif de fermeture comprenant une partie formant corps pouvant tourner (4) incluant un connecteur (6) pour la connexion et la déconnexion avec un connecteur apparié (7) du composant coopérant (8) par rotation relative entre ladite partie formant corps pouvant tourner (4) et le composant coopérant (8), et une partie formant corps relativement stationnaire (12) dudit dispositif de fermeture, ladite partie formant corps pouvant tourner (4) pouvant tourner par rapport à ladite partie formant corps stationnaire (12) entre une position ouverte dans laquelle un passage (20) est défini au travers dudit dispositif de fermeture et une position fermée dans laquelle ledit passage est fermé ; le dispositif de fermeture étant **caractérisé en ce que** lesdites parties formant corps sont construites et agencées de manière que la direction de rotation relative desdits connecteurs pour la connexion de ladite partie formant corps pouvant tourner (4) avec ledit composant coopérant (8) corresponde à la direction de rotation de ladite partie pouvant tourner (4) par rapport à ladite partie formant corps stationnaire (12) en direction de ladite position ouverte pour ouvrir aussi ledit passage (20) et que la direction de rotation relative desdits connecteurs qui déconnectent ladite partie formant corps pouvant tourner (4) d'avec ledit composant coopérant (8) corresponde à la direction de rotation de ladite partie formant corps pouvant tourner (4) par rapport à ladite partie formant corps stationnaire (12) en direction de ladite position fermée pour fermer aussi ledit passage (20).

2. Dispositif de fermeture selon la revendication 1, **caractérisé en ce qu'**il comprend en outre un élément élastique (22), de préférence un section élastique de tube, ayant un axe définissant au moins en partie ledit passage (20) ; **en ce que** ladite partie formant corps pouvant tourner (4) inclut une came interne (18) ayant une surface de came orientée autour de et distante de l'axe dudit passage (20), ladite came ayant une première partie de surface (30) disposée relativement plus près dudit axe qu'une seconde partie de surface (28) écartée suivant la circonférence ; et **en ce qu'**un élément de compression (24) déplaçable radialement est disposé entre et en contact avec ladite surface de came et l'extérieur dudit élément élastique (22) de telle manière que ladite rotation pour la connexion dudit composant (8) avec ledit dispositif de fermeture (2) amène ladite surface de came à ajuster la compression radiale dudit élément élastique (22) plus loin dudit axe pour ouvrir ledit passage (20), et ladite rotation pour déconnecter ledit composant (8) dudit dispositif de fermeture amène ladite surface de came à ajuster la compression radiale dudit élément élastique (22) en direction dudit axe pour fermer ledit passage (20).

3. Dispositif de fermeture selon la revendication 2, **caractérisé en outre en ce que** ladite partie formant corps relativement stationnaire (12) fixe la position dudit tube (22).

4. Dispositif de fermeture selon la revendication 2, **caractérisé en outre en ce que** la force de rotation nécessaire pour faire tourner ladite partie formant corps pouvant tourner (4) pour réaliser un changement de la compression dudit élément élastique (22) pour ouvrir et fermer ledit passage (20) est inférieure à la force de rotation nécessaire, respectivement, pour réaliser la connexion et la déconnexion dudit composant coopérant (8) par rapport audit dispositif (2).

5. Dispositif de fermeture selon la revendication 4, **caractérisé en outre en ce que** ladite partie formant corps pouvant tourner (4) est fixée par vissage autour dudit axe à ladite partie formant corps relativement stationnaire (12), leurs filets (13, 13') étant opposés à la direction de rotation qui permet audit dispositif (2) d'être connecté par rotation audit composant coopérant (8), de sorte que la rotation pour connecter ledit composant coopérant (8) audit dispositif (2) amène lesdites parties formant corps à s'écarter.

6. Dispositif de fermeture selon la revendication 5, **caractérisé en outre en ce qu'**une surface d'arrêt (46) est définie à une extrémité de ladite seconde partie de surface de came (28), ladite surface d'arrêt (46) étant agencée pour entrer en contact avec ledit élément de compression (24) et arrêter le mouvement relatif d'écartement desdites parties formant corps (4, 12) quand ledit élément de compression (24) atteint une position d'ouverture de passage empêchant le désassemblage de ladite partie formant corps par blocage d'une rotation supplémentaire et lesdites parties formant corps pouvant tourner et relativement stationnaire sont construites pour entrer en contact mutuel et arrêter le mouvement de vissage mutuel quand ledit élément de compression atteint une position de fermeture de passage.

7. Dispositif de fermeture selon la revendication 2 ou 4, **caractérisé en outre en ce que** ledit dispositif de fermeture (2) est conçu pour être utilisé comme dispositif de fermeture médicale, ledit passage (20) étant agencé pour le passage d'un fluide sous pression ou d'un dispositif médical dans ou hors d'un corps vivant et l'extrémité proximale (14) dudit dispositif étant construite pour être connectée à un cathéter coopérant ou une seringue.

8. Dispositif de fermeture selon la revendication 7, **caractérisé en outre en ce que** l'extrémité distale (6) dudit dispositif de fermeture est construite pour être connectée à un cathéter, de préférence le cathéter étant moulé d'une pièce avec ladite extrémité distale.

9. Dispositif de fermeture selon la revendication 2, **caractérisé en outre en ce que** ladite première partie de surface (30) de ladite came (18) positionne ledit élément de compression (24), de préférence un élément en forme de bille unique, radialement pour fermer complètement d'une manière étanche aux fluides ledit passage traversant (20) et ladite seconde partie de surface (28) positionne ledit élément de compression pour ouvrir totalement ledit passage traversant (20), ladite came (18) définissant de préférence une surface en forme de spirale régulière.

10. Dispositif selon la revendication 6, **caractérisé en ce qu'**il comprend en outre un filet de vis sur ladite partie formant corps pouvant tourner (4), ledit filet de vis étant configuré pour entrer en contact avec un filet de vis sur ledit composant coopérant (8), et **en ce que** ladite came (18) définit une surface en forme de spirale avec une transition abrupte depuis l'extrémité de ladite seconde partie de surface de came (28), le plus loin de l'axe, jusqu'à une partie de ladite partie formant corps pouvant tourner (4) plus proche de l'axe d'une manière interagissant avec ledit élément de compression (24), ladite manière d'interaction permettant une rotation continuelle pendant l'assemblage de ladite partie formant corps pouvant tourner (4) dans une direction de vissage dans laquelle l'élément de compression (24) est graduellement compressé progressivement contre ledit élément élastique (22) jusqu'au maximum, état dans lequel il reste jusqu'à ce qu'une rotation supplémentaire amène la seconde partie (28) de la surface de came à atteindre ledit élément de compression (24) en permettant audit élément de compression de se détendre vers l'extérieur pour permettre une rotation continuée de ladite partie formant corps pouvant tourner (4), tandis qu'une rotation opposée de ladite partie formant corps pouvant tourner (4) le long desdits filets est limitée par l'arrêt dudit élément de compression (24) contre ladite transition abrupte de ladite partie formant corps pouvant tourner.

11. Dispositif de fermeture selon la revendication 6, **caractérisé en outre en ce que** ledit second arrêt (46) bloque l'avance axiale supplémentaire le long dudit filet de ladite partie formant corps pouvant tourner (4).

12. Dispositif de fermeture selon la revendication 11, **caractérisé en outre en ce que** ladite partie formant corps relativement fixe (12) fixe la position dudit élément élastique (22) et définit une ouverture (26) s'étendant radialement dans laquelle ledit élément de compression (24) est confiné axialement, et est amené à se déplacer radialement contre ledit élément élastique (22).

13. Dispositif de fermeture selon la revendication 2, **caractérisé en outre en ce que** ledit tube (22) est compressé axialement et confiné radialement pour un contact étanche avec les surfaces (42, 38) au niveau de ses extrémités proximale et distale.

14. Dispositif de fermeture selon la revendication 2, **caractérisé en outre en ce que** lesdites parties formant corps et ledit passage (20) sont dimensionnés pour le passage d'un dispositif (20) et fournissent, quand ledit dispositif de fermeture (2) est fermé sur ledit dispositif, des moyens pour saisir et soumettre ledit dispositif à un couple.

15. Dispositif de fermeture selon la revendication 2, **caractérisé en outre en ce que** ledit corps peut être fixé au niveau de ses extrémités proximale et distale à des éléments de tube pour commander l'écoulement du fluide.

16. Dispositif de fermeture selon la revendication 2, **caractérisé en outre en ce que** ladite partie formant corps pouvant tourner (4) a une surface externe exposée pour le contact et l'actionnement en rotation par la main d'un utilisateur.

17. Dispositif de fermeture selon la revendication 2, **caractérisé en outre en ce que** ladite rotation de ladite partie formant corps pouvant tourner (4) pour ouvrir et fermer ledit passage traversant est sensiblement d'un demi-tour.

18. Dispositif de fermeture selon la revendication 1, **caractérisé en outre en ce que** lesdites parties formant corps, en position ouverte, définissent le passage traversant dans lequel un dispositif peut passer.

19. Dispositif de fermeture selon la revendication 18, **caractérisé en outre en ce que** la paroi définissant ledit passage (20) est construite pour fermer et étanchéifier ou saisir un dispositif inséré dans ledit passage traversant.

20. Dispositif de fermeture selon la revendication 7, **caractérisé en ce qu'**il comprend en outre une gaine d'organe d'introduction flexible (120) jointe à l'extrémité distale du dispositif de fermeture, et un cathéter (124) contenant un filtre à veine-cave (105) et un stabilisateur (109) pour la mise en place dudit filtre, ladite gaine d'organe d'introduction (120) et ledit dispositif de fermeture (2a) définissant un passage, de préférence d'une dimension de l'ordre de 1/8 pouce (0,3175 cm) pour faire passer ledit cathéter (124), ladite gaine d'organe d'introduction flexible (120) et ledit dispositif de fermeture (2a) comprenant un ensemble de gaine d'organe d'introduction de cathéter, et ledit cathéter (124) et ledit ensemble de gaine d'organe d'introduction de cathéter étant contenus dans un kit d'organe d'introduction de cathéter.

21. Dispositif de fermeture selon la revendication 20, **caractérisé en outre en ce que** ladite partie formant corps pouvant tourner (4) est fixée par vissage autour dudit axe à ladite partie formant corps relativement stationnaire (12) et ledit élément de compression (24) est ajustable par manipulation manuelle de ladite partie formant corps pouvant tourner (4) jusque dans des positions pouvant tourner intermédiaires choisies par rapport à ladite partie formant corps relativement stationnaire (12) pour amener ledit élément élastique (22) à venir en contact légèrement avec les côtés dudit cathéter (124) qui est inséré dans ledit ensemble de gaine d'organe d'introduction de cathéter pour entraver un écoulement de sang vers l'extérieur le long des côtés dudit cathéter tout en permettant son insertion à coulissement.

22. Dispositif de fermeture selon la revendication 20, **caractérisé en outre en ce qu'**il comporte un dilatateur allongé amovible (122) disposé dans ladite gaine d'organe d'introduction flexible (120), ledit dilatateur (122) ayant une partie distale effilée (126) s'étendant distalement au-delà de la gaine d'organe d'introduction flexible.

23. Dispositif de fermeture selon la revendication 23, **caractérisé en outre en ce que** ledit dilatateur (122) a un petit passage axial permettant audit dilatateur (122), à la gaine d'organe d'introduction flexible (120) et au dispositif de fermeture (2a) d'être enfilés sur un fil de guidage placé au préalable pour guider la gaine (122) jusqu'à une position prédéterminée.

24. Dispositif de fermeture médical pour former un passage (20) dans ou hors d'un composant coopérant médical (8), le composant coopérant médical (8) étant conçu pour être inséré dans un corps humain ou animal, le dispositif de fermeture médical formant un passage pour des fluides sous pression et un dispositif médical, ledit dispositif de fermeture médical étant **caractérisé en ce qu'**il comprend : un dispositif de fermeture selon la revendication 1, le connecteur (6) de la partie formant corps pouvant tourner (4) qui est adapté pour la connexion et la déconnexion avec un connecteur apparié (7) dudit composant coopérant médical (8).

25. Dispositif de fermeture médical selon la revendication 24, **caractérisé en ce qu'**il comprend en outre : un élément élastique (22) définissant au moins en partie ledit passage (20), ledit passage ayant un axe et étant agencé pour le passage d'un dispositif médical et d'un fluide sous pression choisis, ledit élément élastique (22) étant en un matériau choisi, en réponse à une pression radiale appliquée audit élément élastique (22), pour former un joint d'étanchéité par pression sur ledit dispositif médical quand ledit dispositif médical est présent dans ledit passage (20) et sur une surface opposée définissant ledit passage quand ledit dispositif médical n'est pas présent ; une partie formant corps proximale (12) incluant les premier et second éléments, ledit premier élément ayant un raccord par pression en contact d'étanchéité avec et portant ledit élément (22) et ledit second élément étant disposé autour de et soutenant ledit élément élastique (22) ; une partie formant corps distale (4) incluant une came interne (18) ayant une surface de came orientée autour de et distante de l'axe dudit passage (20) avec une première surface de came (30) disposée relativement plus près dudit axe qu'une seconde surface (28) distante suivant la circonférence ; et un élément de compression (24) positionné dans une ouverture (26) s'étendant radialement dans ladite partie formant corps proximale (12) et déplacé radialement vers l'extérieur par ledit élément élastique (22) pour maintenir un contact avec ladite surface de came ; et étant **caractérisé en outre en ce que** lesdites parties formant corps proximale et distale (4, 12) sont connectées mutuellement d'une manière permettant leur rotation relative autour dudit axe, lesdites parties formant corps (4, 12) et ledit élément élastique (22) étant étanchéifiés ensemble de manière à résister, sans fuite, à un fluide sous pression dans ledit passage ; **en ce que** lesdites parties formant corps connectées (4, 12) sont reliées en coopération, de sorte que, lors d'une rotation relative desdites parties formant corps (4, 12), ladite surface de came est déplacée par rapport audit élément de compression (24) pour entraîner un déplacement radial dudit élément de compression (24) plus près de l'axe dudit passage pour amener ledit élément élastique (22) à saisir et former un joint d'étanchéité par pression sur l'extérieur dudit dispositif médical, quand il est présent dans ledit passage, ou sur ladite surface opposée définissant ledit passage quand ledit dispositif médical n'est pas présent.

26. Dispositif de fermeture selon la revendication 14, **caractérisé en outre en ce que** ledit dispositif comprend en outre un élément d'étanchéité (210) monté sur ladite seconde partie de ladite partie formant corps proximale (12) pour améliorer le contact d'étanchéité entre lesdites parties formant corps distale et proximale (4, 12).

27. Procédé d'ouverture ou de fermeture d'un passage par lequel des fluides sous pression et un dispositif médical peuvent passer dans ou hors du corps, comprenant les étapes de : connexion ou déconnexion d'un composant coopérant (8) par rapport à ladite partie formant corps pouvant tourner (4) d'un dispositif de fermeture selon la revendication 1 pour ouvrir ou fermer ledit passage, respectivement, par une rotation relative entre ladite partie formant corps pouvant tourner (4) et le composant coopérant (8).

28. Procédé selon la revendication 27, comprenant en outre les étapes de : introduction d'un dispositif médical dans ledit passage ; et, tandis que ledit dispositif est dans ledit passage, rotation relative de ladite seconde partie formant corps dans la direction qui déconnecte ledit composant dudit dispositif de fermeture pour amener un élément élastique (22) à l'intérieur dudit dispositif de fermeture à saisir et former un joint d'étanchéité par pression sur l'extérieur dudit dispositif.

29. Procédé selon la revendication 27, où ledit composant coopérant est une seringue pour fournir des fluides sous pression.
